(19) **Europäisches Patentamt**

**European Patent Office**

**Office européen des brevets**

(11) **EP 1 416 281 A2**

(12) **EUROPEAN PATENT APPLICATION**

(43) Date of publication:
06.05.2004 Bulletin 2004/19

(51) Int Cl.$^7$: **G01N 33/68**, C07K 14/47

(21) Application number: **04000945.8**

(22) Date of filing: **20.02.1998**

(84) Designated Contracting States:
**AT BE CH DE DK ES FI FR GB GR IE IT LI LU MC NL PT SE**

(30) Priority: **21.02.1997 US 804536**

(62) Document number(s) of the earlier application(s) in accordance with Art. 76 EPC:
**98906471.2 / 0 970 372**

(71) Applicant: **The Regents of the University of California**
**Oakland, CA 94607-5200 (US)**

(72) Inventors:
• **Prusiner, Stanley B.**
**San Francisco California 94116 (US)**

• **Safar, Jiri G.**
**Concord California 94518 (US)**

(74) Representative: **Campbell, Patrick John Henry**
**J.A. Kemp & Co.,**
**14 South Square,**
**Gray's Inn**
**London WC1R 5JJ (GB)**

Remarks:
This application was filed on 19 - 01 - 2004 as a divisional application to the application mentioned under INID code 62.

(54) **Assay for disease related conformation of a protein**

(57) The invention provides an assay method for determining the presence of a PrP$^{Sc}$ form of a PrP protein in a sample comprising a first PrP$^C$ conformation of the protein and a second PrP$^{Sc}$ conformation of the protein, the method comprising contacting a first portion of the sample with a binding partner, said binding partner having a higher affinity for the PrP$^C$ conformation than the PrP$^{Sc}$ conformation, and determining a first concentration; treating a second portion of the sample to change the PrP$^{Sc}$ conformation into a conformation with a higher binding affinity for the binding partner; contacting the treated second portion of the sample with the binding partner to determine a second concentration; adjusting the second concentration to provide an adjusted concentration which adjustment compensates for increased affinity of the PrP$^C$ conformation for the binding partner resulting from the treating; and comparing the first concentration with the adjusted concentration to determine the presence of protein in the PrP$^{Sc}$ conformation.

**Description**

FIELD OF THE INVENTION

This invention relates generally to immunoassays. More particularly the invention relates to an assay which allows for detection of a disease related conformational form of a protein (such as PrP^Sc) which may have very low antibody binding affinity and further allows identification of the particular strain responsible for the disease.

BACKGROUND OF THE INVENTION

Prions are infectious pathogens that cause invariably fatal prion diseases (spongiform encephalopathies) of the central nervous system in humans and animals. Prions differ significantly from bacteria, viruses and viroids. The dominating hypothesis is that no nucleic acid is necessary to allow for the infectivity of a prion protein to proceed.

A major step in the study of prions and the diseases they cause was the discovery and purification of a protein designated prion protein [Bolton, McKinley et al. (1982) Science 218:1309-1311; Prusiner, Bolton et al. (1982) Biochemistry 21:6942-6950; McKinley, Bolton et al. (1983) Cell 15:57-62]. Complete prion protein-encoding genes have since been cloned, sequenced and expressed in transgenic animals. PrP^c is encoded by a single-copy host gene [Basler, Oesch et al. (1986) Cell 46 :417-428] and when PrP^c is expressed it is generally found on the outer surface of neurons. Many lines of evidence indicate that prion diseases results from the transformation of the normal form of prion protein (PrP^c) into the abnormal form (PrP^Sc). There is no detectable difference in the amino acid sequence of the two forms. However, PrP^Sc when compared with PrP^c has a conformation with higher β-sheet and lower α-helix content [Pan, Baldwin et al. (1993) Proc Natl Acad Sci USA 90:10962-10966; Safar, Roller et al. (1993) J Biol Chem 268:20276-20284]. The presence of the abnormal PrP^Sc form in the brains of infected humans or animals is the only disease-specific diagnostic marker of prion diseases.

PrP^Sc plays a key role in both transmission and pathogenesis of prion diseases (spongiform encephalopathies) and it is a critical factor in neuronal degeneration [Prusiner (1997) The Molecular and Genetic Basis of Neurological Disease, 2nd Edition : 103-143]. The most common prion diseases in animals are scrapie of sheep and goats and bovine spongiform encephalopathy (BSE) of cattle [Wilesmith and Wells (1991) Curr Top Microbiol Immunol 172: 21-38]. Four prion diseases of humans have been identified: (1) kuru, (2) Creutzfeldt-Jakob Disease (CJD), (3) Gerstmann-Streussler-Sheinker Disease (GSS), and (4) fatal familial insomnia (FFI) [Gajdusek (1977) Science 197: 943-960; Medori, Tritschler et al. (1992) N Engl J Med 326:444-449]. Initially, the presentation of the inherited human prion diseases posed a conundrum which has since been explained by the cellular genetic origin of PrP.

Prions exist in multiple isolates (strains) with distinct biological characteristics when these different strains infect in genetically identical hosts [Prusiner (1997) The Molecular and Genetic Basis of Neurological Disease, 2nd Edition: 165-186]. The strains differ by incubation time, by topology of accumulation of PrP^Sc protein, and in some cases also by distribution and characteristics of brain pathology [DeArmond and Prusiner (1997) Greenfield's Neuropathology, 6th Edition:235-280]. Because PrP^Sc is the major, and very probably the only component of prions, the existence of prion strains has posed a conundrum as to how biological information can be enciphered in a molecule other than one comprised of nucleic acids. The partial proteolytic treatment of brain homogenates containing some prion isolates has been found to generate peptides with slightly different electrophoretic mobilities [Bessen and Marsh (1992) J Virol 66:2096-2101; Bessen and Marsh (1992) J Gen Virol 73:329-334; Telling, Parchi et al. (1996) Science 274:2079-2082]. These findings suggested different proteolytic cleavage sites due to the different conformation of PrP^Sc molecules in different strains of prions. Alternatively, the observed differences could be explained by formation of different complexes with other molecules, forming distinct cleavage sites in PrP^Sc in different strains [Marsh and Bessen (1994) Phil Trans R Soc Lond B 343:413-414]. Some researchers have proposed that different prion isolates may differ in the glycosylation patterns of prion protein [Collinge, Sidle et al. (1996) Nature 383:685-690; Hill, Zeidler et al. (1997) Lancet 349:99-100]. However, the reliability of both glycosylation and peptide mapping patterns in diagnostics of multiple prion strains is currently still debated [Collings, Hill et al. (1997) Nature 386:564; Somerville, Chong et al. (1997) Nature 386:564].

A system for detecting PrP^Sc by enhancing immunoreactivity after denaturation is provided in Serban, et al., Neurology, Vol. 40, No. 1, Ja 1990. Sufficiently sensitive and specific direct assay for infectious PrP^Sc in biological samples could potentially abolish the need for animal inoculations completely. Unfortunately, such does not appear to be possible with current PrP^Sc assays -- it is estimated that the current sensitivity limit of proteinase-K and Western blot-based PrP^Sc detection is in a range of 1μg/ml which corresponds to $10^4 - 10^5$ prion infectious units. Additionally, the specificity of the traditional proteinase-K-based assays for PrP^Sc is in question in light of recent findings of only relative or no proteinase-K resistance of undoubtedly infectious prion preparations [Hsiao, Groth et al. (1994) Proc Natl Acad Sci USA 91:9126-9130] Telling, et al. (1996) Genes & Dev.

Human transthyretin (TTR) is a normal plasma protein composed of four identical, predominantly β-sheet structured units, and serves as a transporter of hormone thyroxin. Abnormal self assembly of TTR into amyloid fibrils causes two forms of human diseases, namely senile systemic amyloidosis (SSA) and familial amyloid polyneuropathy (FAP) [Kelly (1996) Curr Opin Strut Biol 6(1): 11-7]. The cause of amyloid formation in FAP are point mutations in the TTR gene; the cause of SSA is unknown. The clinical diagnosis is established histologically by detecting deposits of amyloid in situ in bioptic material.

To date, little is known about the mechanism of TTR conversion into amyloid in vivo. However, several laboratories have demonstrated that amyloid conversion may be simulated *in vitro* by partial denaturation of normal human TTR [McCutchen, Colon et al. (1993) Biochemistry 32(45):12119-27; McCutchen and Kelly (1993) Biochem Biophys Res Commun 197(2) 415-21]. The mechanism of conformational transition involves monomeric conformational intermediate which polymerizes into linear β-sheet structured amyloid fibrils [Lai, Colon et al. (1996) Biochemistry 35(20): 6470-82]. The process can be mitigated by binding with stabilizing molecules such as thyroxin or triiodophenol [Miroy, Lai et al. (1996) Proc Natl Acad Sci USA 93(261:15051-6].

In view of the above points, there is clearly a need for a specific, high flow-through, and cost-effective assay for testing sample materials for the presence of pathogenic protein including transthyretin and prion protein. The presented invention offers an assay capable not only of detecting pathogenic proteins but of determining the specific strain.

SUMMARY OF THE INVENTION

There are two different procedures for detecting low levels of a disease conformation of a protein. The simplest method requires that a predetermined standard has been calculated, and comprises providing a sample suspected of containing a protein which assumes two conformations (a first conformation, and a second, disease-related conformation), treating the sample to convert any protein in the second conformation into a different conformation which has a higher antibody binding affinity, contacting the treated sample with an antibody which binds to the protein in its first conformation and/or the different conformation with a higher affinity than to the second conformation, determining the level of binding of antibody to protein, and comparing the level of binding to the predetermined standard thereby determining the probability of the sample containing protein in the second, disease-related conformation based on the comparison. The methodology disclosed here allows for the detection of the disease conformation at a level of $1 \times 10^3$ particles/ml or less.

The assay of the invention can also be conducted without using a predetermined standard. This assay method comprises (a) providing a sample suspected of containing a protein (having a first conformation and a second, disease-related conformation), (b) dividing the sample into first and second portions, (c) contacting the first portion with an antibody that binds to the first conformation with higher affinity than to the second conformation, (d) treating the second portion to cause any protein in the second conformation to adopt a different conformation having a higher affinity for the antibody, (e) contacting the second portion with the antibody, (f) determining the relative levels of antibody binding to said first and second portions, and (g) determining the presence or absence of protein in the second conformation based on the comparison.

The assay of the invention is useful in assaying samples which contain proteins which proteins are present in at least two conformations (e.g., a native non-disease conformation and a disease conformation) and are present at levels of $1 \times 10^3$ particles/ml or less. The present invention utilizes antibodies which do not bind or have a relatively low degree of affinity for the tightly configured disease-conformation of the protein. One useful antibody is the monoclonal antibody 263K 3F4 produced by the hybridoma cell line ATCC HB9222 deposited on October 8, 1986 in the American Type Culture Collection, 12301 Parklawn Drive, Rockville, MD 20852 and disclosed and described in U.S. Patent 4,806,627 issued February 21, 1989 - incorporated by reference to disclose antibodies which selectively bind PrP[c].

A specific example of the assay may be carried out by providing a sample which is divided into a first portion and a second portion. The first portion is bound to a first solid support and then contacted with a labeled antibody which binds to the non-disease form of the protein with a higher degree of affinity (e.g., 4 to 30 fold higher affinity) than the antibody binds to the disease form. The second portion of the sample is treated in a manner which causes the tightly bound disease form of the protein present in the sample (if any) to assume a more relaxed conformation which has a higher binding affinity to the labeled antibody. After treatment the second portion is also bound to the surface of a solid support. Thereafter, the second portion is contacted with the same type of labeled antibodies which were used on the first portion. Based on the amount of antibody binding protein on the support, the level of protein in the first portion is then determined. The level of protein in the second treated portion is determined in the same manner. The difference between the two is determined and if it is found that the amount of binding of antibodies to proteins in the second portion is significantly higher than that of the first portion it is possible to deduce that the original sample contained proteins in the tightly bound disease related conformation. Further, by use of formulae and a particularly sensitive assay system provided herein it is possible to determine the amount of the disease related conformation of the protein present in the original sample per unit of volume. Still further, by determining the ratio of antibody binding to denatured protein to antibody binding to native protein, it is possible to determine the particular strain of a disease related protein present.

To demonstrate the basic concept behind the present invention it is necessary to include a starting sample which is divided into at least two portions. The first portion is contacted with labeled antibodies without treating the proteins and the second portion is treated with labeled antibodies after the proteins have been treated in a manner which causes any proteins in the disease conformation to assume a conformation which has a higher degree of binding affinity for the antibodies. The readings are compared (i.e., one subtracted from the other) and the presence of proteins in the disease related conformation are deduced based on the difference between the two readings. However, it is possible to utilize the basic concept behind the present invention without obtaining two readings for each assay. This can be done by establishing a standard based on carrying out the assay on a statistically significant number of closely related samples. After the standard has been established one will know the level of antibody binding which should be observed when the sample does not contain any proteins in the disease related conformation. Using the standard, one then treats a sample to be tested so as to convert any proteins in the disease related conformation to a different conformation which has a much higher degree of binding affinity for the label antibodies. The measurement obtained is then compared with the standard. If the difference between the standard and the measurement obtained is outside of a given range it can be deduced that the original sample included proteins in the disease related conformation.

A third embodiment of the invention can utilize either of the embodiments disclosed above along with the formulae provided herein in order to calculate (quantitatively) the number of proteins in the disease related conformation present within the original sample.

In a fourth embodiment, the particular strain of infectious protein present in a sample is determined. The strain is determined by matching the "protein index" of the sample tested with the known protein index of a particle strain of a given protein. The "protein index" of the sample is calculated by determining the ratio of the amount of antibody which binds the denatured form of the protein to the amount of antibody which binds to the native protein.

In accordance with any of the systems it is preferable to pre-treat the sample being tested in a manner which decreases the concentration of the non-disease conformation relative to the concentration of the disease conformation of the protein. For example, the initial sample can be chemically treated with a compound which preferentially degrades the relaxed, non-disease form of the protein and/or is exposed to antibodies which preferentially bind to and thereby remove the non-disease conformation of the protein.

It may be possible to further enhance the sensitivity of various aspects of the invention by concentrating the disease conformation of a protein by adding a compound which selectively binds to the disease conformation to form a complex and centrifuging the sample to precipitate out the complex which is then tested in accordance with the methods described here. Specifics regarding such concentration methods are described in detail in our co-pending application attorney docket number 6510/098001 filed on the same date as the present application entitled "Process for Concentrating Protein with Disease-Related Conformation".

The different embodiments of the assay of the invention described above are all "direct" types of immunoassays -- meaning that the sample is directly assayed with the labeled antibody either with or without treatment to change the conformation of any disease related conformation proteins present in the sample. An "indirect" assay may also be used. For example, it may be desirable to enhance the number of disease related proteins in the sample (if any) by the use of a transgenic mouse and thereby enhance any signal obtained. To carry out these embodiments of the invention, the sample is first used to inoculate a transgenic mouse which has had its genome modified so that it will develop symptoms of disease when inoculated with proteins in the disease related conformation. After the mice are inoculated, a sufficient period of time is allowed to pass (e.g., 30 days) after which the transgenic animal is sacrificed and a sample such as homogenized brain tissue from the mouse is used in the direct assay described above. The present invention enhances the ability of transgenic mice to detect prions by shortening the period of time which must pass until a determination can be made as to whether the original sample included proteins in the disease related conformation. It would also be possible to apply epitope tagged PrP as disclosed in pending U.S. patent application Serial No. 08/660,626, filed June 6, 1996 (incorporated by reference) to affinity purify the PrP$^{Sc}$ from the brain of a Tg mouse and thereafter apply the assay of the present invention. Without the present invention the mouse is inoculated and one must wait until the inoculated mouse actually demonstrates symptoms of the disease. Depending on the mouse, this can take several months or even years.

The assay methodology of the present invention can be applied to any type of sample when the sample is suspected of containing a protein which occurs in at least two conformations. The protein must occur in one conformation which binds to known antibodies, antibodies which can be generated or other specific binding partners. The second conformation must be sufficiently different from the first conformation in terms of its binding affinity so that the two conformations can be distinguished by using antibodies or binding partners which have a much higher degree of affinity for the first conformation than for the second conformation. In its conceptually simplest form, the invention works best when a known labeled antibody binds to a non-disease form of a protein with a high degree of affinity, and does not bind (or binds with an extremely low degree of affinity) to the same protein when it is present in its disease related conformation. However, in reality, a given protein may have more than two conformations. The protein may have more than one non-disease conformation and more than one disease related conformation, (Telling, et al., Science (1996)). The invention is still useful when multiple conformations of non-disease and disease forms of the protein exist - provided that (1) at least one non-disease conformation differs from at least one disease conformation in terms of its binding affinity; and (2) it is possible to treat the disease related conformation of the protein so as to substantially enhance its binding affinity.

As indicated above, the assay of the invention can be used to assay any type of sample for any type of protein, provided the protein includes a non-disease and a disease related conformation. However, the invention was particularly developed to assay samples for the presence of (1) PrP proteins and determine whether the sample included a PrP protein in its disease conformation, i.e., included PrP$^{Sc}$ (2) insoluble forms of βA4 associated with Alzheimer's disease and (3) transthyretin. Accordingly, much of the following disclosure is directed to using the immunoassay of the present invention to detect the presence of either PrP$^{Sc}$ (or to a lesser degree βA4 or transthyretin (TTR)) in a sample - it being understood that the same general concepts are applicable to detecting disease related conformations of a wide range of different types of proteins. Further, the disclosure is particularly directed to describing how to determine the particular strain of infectious prions (PrP$^{Sc}$) in a sample - it being understood that the same general concepts are applicable to determining the particular strain of other constricted proteins associated with different diseases.

The present method of PrP$^{Sc}$ detection was developed by labeling selected purified IgG with Europium. Antibodies used have a high binding affinity for PrP$^{c}$ (non-disease conformation) which comprises an α-helical configuration. The antibodies have a low binding affinity for PrP$^{Sc}$ (disease conformation) which comprises a β-sheet configuration. The IgG may be obtained from common monoclonal, polyclonal, or recombinant antibodies, typically recognizing the sequence 90-145 or 222-231 of PrP$^{Sc}$ and conformationally unfolded prion protein. Different conformations of recombinant prion protein were chemically crosslinked to polystyrene plates through a glutaraldehyde activation step. The relative affinities of the Eu-labeled IgG with α-helical, β-sheet, and random coil conformation of recombinant Syrian hamster prion protein corresponding to sequence 90-231 were determined by time-resolved, dissociation-enhanced fluorescence in a 96-well polystyrene plate format.

A determination of the relative affinities of different labeled antibodies for proteins can be made by different methods. However, the disease related conformation of a protein is often present in a very low concentration relative to that of the non-disease conformation. Accordingly, it often requires very sensitive methods to detect any increase caused by the treatment of the disease conformation of the protein. One particularly sensitive method is time-resolved, dissociation-enhanced fluorescence.

By carefully calibrating this method, it is possible to detect the signal increase in antibody reactivity in the transition from β-sheet conformational state to denatured state. This signal is relatively large compared to that obtained from the transformation from its native α-helical to the treated relaxed, or denatured state. Thus, the original conformational state of prion protein can be assigned by the differential assay in native and treated states. When a sample containing no β-sheet protein is treated some increase is obtained in immunoreactivity. This amount of increase must be adjusted for and after doing such the resulting concentration or amount is referred to the "adjusted amount." The amount of antibody-specific binding over that obtained for α-helical conformation of PrP$^{c}$ (beyond the adjusted amount) is a measure of the presence of β-sheet conformation which is essential for pathogenicity and infectivity of PrP$^{Sc}$.

A primary object of the invention is to provide an immunoassay which is applicable to assaying samples containing proteins, which samples are suspected of containing a protein which occurs within a native non-disease conformation and a disease related conformation (e.g., PrP protein, βA4 protein and transthyretin).

An advantage of the present invention is that the immunoassay can quickly and accurately determine the presence of proteins in the disease related conformation (e.g., PrP$^{Sc}$, βA4 and transthyretin) even though the antibody used in the assay does not bind or has a very low degree of binding affinity for the protein in the disease related conformation and the disease related conformation is present in a lower concentration than the non-disease conformation.

Another object of the invention is to provide an assay which makes it possible to not only determine (1) whether a pathogenic particle is present in a sample but (2) determine the concentration of the particles in a sample, and (3) determine the particular strain of particle present.

A feature of the invention is that the signal obtained can be enhanced by the use of transgenic animals, e.g., mice which are used to detect the presence of a protein in a sample.

Another feature is that time-resolved, dissociation-enhanced fluorescence is used to enhance sensitivity.

Another advantage is that the assay can detect levels of disease conformation of a protein at a concentration of $1 \times 10^3$ particles/ml or less.

A specific object is to provide a diagnostic assay for determining the presence of infectious prion protein in variable sample materials obtained or derived from human, primate, monkey, pig, bovine, sheep, deer, elk, cat, dog, mouse, and chicken tissues and/or body fluids.

Another specific object is to provide a diagnostic assay for determining the presence of βA4 protein in variable sample materials obtained or derived from human, primate, monkey, pig, bovine, sheep, deer, elk, cat, dog, mouse, and chicken tissues and/or body fluids.

Another object is to provide a rapid assay for native infectious prion protein in the brains of transgenic and non-transgenic animals injected with sample material potentially containing prions.

Another object is to provide a method to evaluate decontamination procedures by assaying the level of denaturation of pathogenic proteins (e.g., prions or $\beta$-sheet $\beta$A4) after such treatments.

Another object is to provide a rapid method for screening different compounds to evaluate their potential for treating diseases associated with disease conformations of different proteins such as by screening compounds for their stabilizing effect on different protein conformations (e.g., PrP$^c$ or $\alpha$-helical conformation of $\beta$A4) or their destabilizing impact on the pathogenic conformation (e.g., PrP$^{Sc}$ or $\beta$-sheet conformation of $\beta$A4) of a protein.

Another object is to provide a rapid method for screening different pharmaceutical compounds with potential for prion disease treatment such as by screening compounds for their stabilizing effect on $\alpha$-helical conformation of a normal isoform of the PrP$^c$ protein or their destabilizing impact on the $\beta$-sheet conformation of the pathogenic isoform of the PrP$^{Sc}$ protein.

Another advantage is that the process can be carried out without an antibody directly able to recognize an infectious conformation of a protein, and without using a proteinase K step to eliminate the signal of normal (non-disease) isoforms of the protein such as PrP$^c$.

Another advantage is that in the invented process there is no need for the antibody directly able to recognize pathogenic conformation of $\beta$A4 or transthyretin.

An important feature of the assay is the rapid, cost effective and high flow-through design which can be designed with the capacity to screen 96 samples per day per 96 well plate.

Another aspect of the invention is the diagnostic method to quantitatively detect TTR in the abnormal, amyloid conformation in sample material obtained from human and animal tissues, body fluids, and pharmaceuticals. The invented process provides a direct, sensitive method to distinguish and quantify the normal and amyloid conformations of TTR in a mixture present in sample materials.

The quantitation is based on a measurement of the difference in affinities of monoclonal or polyclonal antibodies with TTR in normal or amyloid conformation against random coil conformation. The present invention describes three methods of evaluation and the mathematical formula used for such quantification.

An important object is to provide specific diagnostic assay for pathogenic TTR in variable sample materials obtained or derived from human, primate, monkey, pig bovine, sheep, deer, elk, cat, dog, and chicken tissues.

Another object is to provide a rapid assay for amyloid form of TTR in transgenic animals.

Another object is to provide a rapid method to screen different pharmaceutical compounds with potential for treatment of senile systemic amyloidosis (SSA) and familial arnyloidotic polyneuropathy (FAP). Such compounds are screened for their stabilizing effect on normal conformation of TTR or their destabilizing impact on the amyloid conformation of TTR.

Still another object is to provide a rapid method to screen the impact of different spontaneous and designed mutations in the TTR gene on conformation, stability and amyloid formation of such TTR gene products in transgenic animals harboring natural or artificial APP genes.

The specific advantage is that invented assay may detect a pathogenic forms of TTR in a mixture with denatured nonpathogenic forms of the same or in a mixture with a soluble form of TTR - for example, detect less than 1 x $10^3$ particles per ml.

These and other objects, advantages, and features of the invented process will become apparent to those skilled in the art upon reading the details of the assay method, antibody development and testing, and transgenic mouse as more fully described below with reference to the attached figures.

BRIEF DESCRIPTION OF THE DRAWINGS

Figure 1 is a spectrograph of the conformation of recombinant SHaPrP90-231 as determined by circular dichroism (CD) spectroscopy showing the two major bands with minima at 208 and 222 nm indicate $\alpha$-helical conformation; single negative band with minimum at 217 nm is characteristic of predominantly $\beta$-sheet conformation;

Figure 2 is a graph showing the results of competitive assay of recombinant SHaPrP90-231 in $\alpha$-helical and denatured conformations in the presence of 5% PrP$^{0/0}$ mouse brain homogenate wherein the difference in slope and crossover points obtained with Europium-labeled 3F4 IgG indicate that each conformations has both a different affinity and number of binding sites and further wherein the data points and bars represent average±SEM obtained from four independent measurements;

Figure 3 is a graph showing the calibration of a direct assay with recombinant SHaPrP90-231 in $\alpha$-helical conformation, in the presence of 5% PrP$^{0/0}$ mouse brain homogenate wherein the data points and bars represent average±SEM obtained from four independent measurements;

Figure 4 is a graph showing the calibration of a direct assay with recombinant SHaPrP90-231 in β-sheet conformation, in the presence of 5% PrP$^{0/0}$ mouse brain homogenate wherein the data points and bars represent average± SEM obtained from four independent measurements;

Figure 5 is a graph showing the input-output validation of a direct assay for both α-helical and β-sheet forms of SHaPrP90-231 in the presence of 5% PrP$^{0/0}$ mouse brain homogenate wherein the amount of the protein on the x axis was determined by amino acid analysis and the amount of the protein on the y axis is calculated from the assay;

Figure 6 is a graph showing the ratio between the signals of treated (denatured) and native SHaPrP90-231 in α-helical and β-sheet conformations, developed with Eu-labeled 3F4 IgG wherein the data points and bars represent average ± SEM obtained from four independent measurements;

Figure 7 is a graph showing the results of a direct assay for PrP$^c$ protein in normal hamster brain homogenate wherein the data points and bars represent average ± SEM obtained from four independent measurements;

Figure 8 is a graph showing the results of a direct assay for PrP$^{c+Sc}$ in scrapie infected hamster brain homogenate wherein the data points and bars represent average±SEM obtained from four independent measurements;

Figure 9 is a graph showing the total amount of PrP proteins in normal hamster brains and the amount of β-sheet PrP$^{Sc}$ in both brains calculated from the model wherein the data points and bars represent average ± SEM obtained from four independent measurements;

Figure 10 is a graph showing the total amount of PrP proteins in scrapie infected hamster brains and the amount of β-sheet PrP$^{Sc}$ in both brains calculated from the model wherein the data points and bars represent average±SEM obtained from four independent measurements;

Figure 11 is a graph showing the correlation of the infectivity and amount of β-sheet form of SHaPrP$^{Sc}$ as calculated from the direct assay and formula wherein purified SHaPrP$^{Sc}$ was sonicated in the presence of 5% PrP$^{0/0}$ mouse brain homogenate and diluted as described and wherein the data points and bars represent average±SEM obtained from four independent measurements.

Figure 12 is a graph of βA4 (1-40) in both α-helical and β-sheet conformations produced by circular dichroism (CD) spectroscopy showing major bonds at 208 and 222 nm for the helical conformation and a single negative band at 217 nm for the predominantly β-sheet conformation (at pH 7.4);

Figure 13 is a graph of a direct assay of soluble A4β (1-40);

Figure 14 is a graph of a direct assay of the β-sheet form of A4β (1-40);

Figure 15 is a graph showing the ratio of denature to native A4β (1-40);

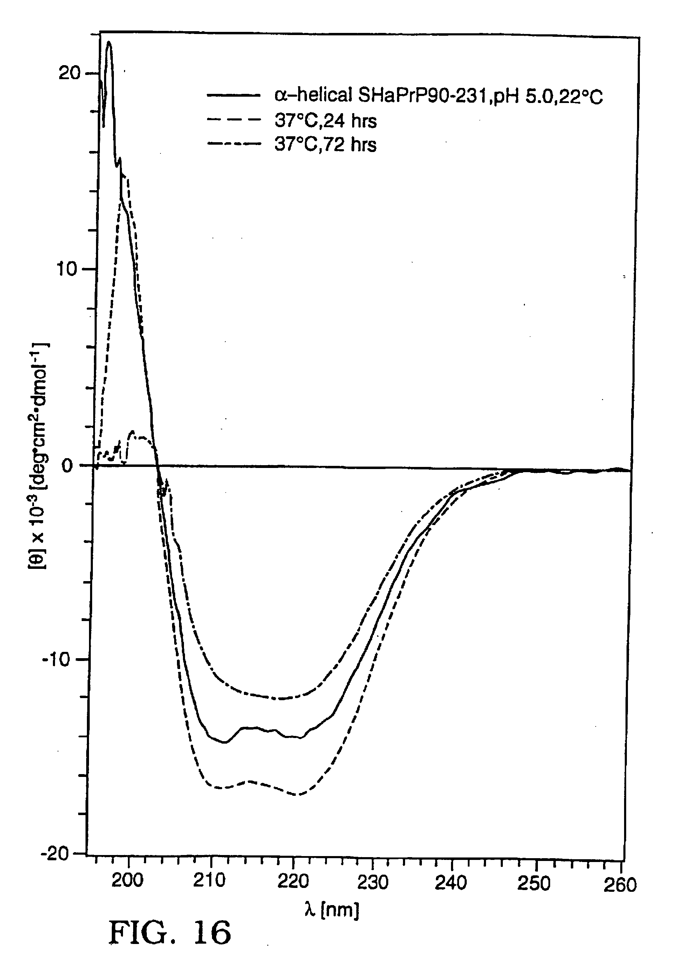

Figure 16 shows the conversion of recombinant ShaPrP90-231 from α-helical to β-sheet conformation during incubation at 37°C for 72 hrs, as determined by circular dichroism (CD) spectroscopy. The protein concentration was 5 mg/ml;

Figure 17 shows conversion of recombinant ShaPrP90-231 from α-helical to β-sheet conformation during incubation at 37°C for 72 hrs, as determined by direct differential assay. The increased signal of native conformation at ~24 hrs indicates destabilization of native structure with more open conformation followed by conversion to β-sheet secondary structure (see Figure 16). The protein concentration was 5 mg/ml;

Figure 18 shows that both HFIP and glycerol are able to prevent α-to-β conformational transition of recombinant ShaPrP90-231. The changes in the TRF signal are expressed as fractional change, where positive value indicates stabilization, negative destabilization. The experimental conditions were as in Fig. 16 and 17;

Figure 19: Pentosan polysulphate stabilizes native conformation of ShaPrP90-231 at low concentrations; Congo red has no effect on stability of ShaPrP90-231. The changes in the TRF signal are expressed as fractional change, where positive value indicates stabilization, negative destabilization. The experimental conditions were as in Figs. 16 and 17;

Figure 20: Zwitterionic detergent ZW3-12 destabilized native conformation of α-helical Sha90-231. The experimental conditions were as in Fig. 16 and 17; the changes in the TRF signal are expressed as fractional change, where positive value indicates stabilization, negative destabilization;

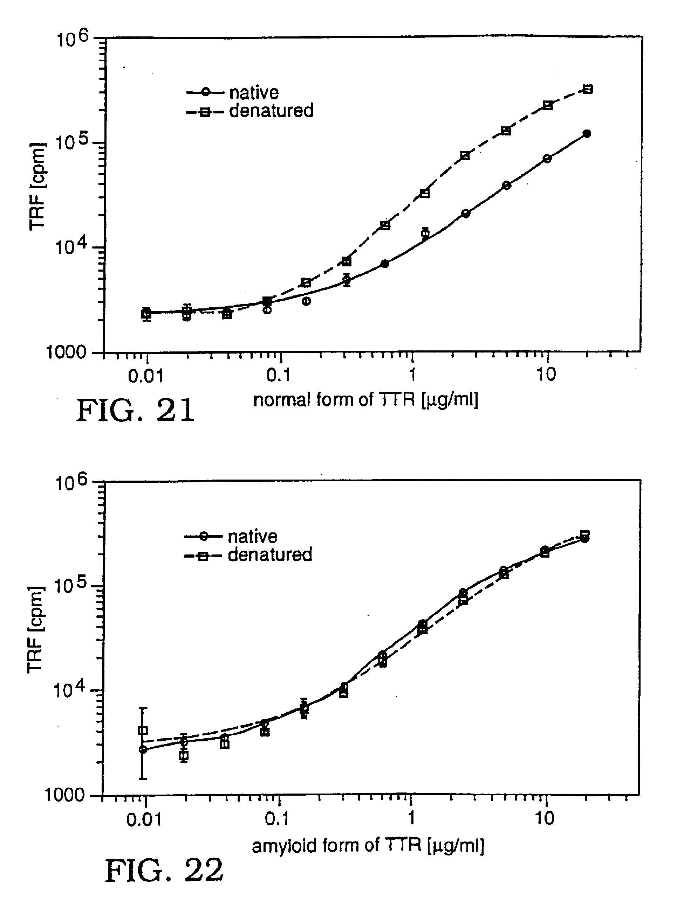

Figure 21 shows calibration of a direct assay with purified human TTR in normal conformation. The plates were developed with the anti-TTR primary antibodies (Accurate Chemical and Scientific Corporation, Westbury, NY) and secondary Eu-labeled anti-rabbit antibody. The data points and bars represent average ± SEM obtained from four independent measurements;

Figure 22 shows calibration of direct assay with purified human TTR in amyloid conformation. The plates were developed with anti-TTR primary antibodies and secondary Eu-labeled anti-rabbit antibody. The data points and bars represent average ± SEM obtained from four independent measurements;

Figure 23 shows the ratio between signals of denatured and native TTR in normal and amyloid conformations, developed as described above. The data points and bars represent average± SEM obtained from four independent measurements;

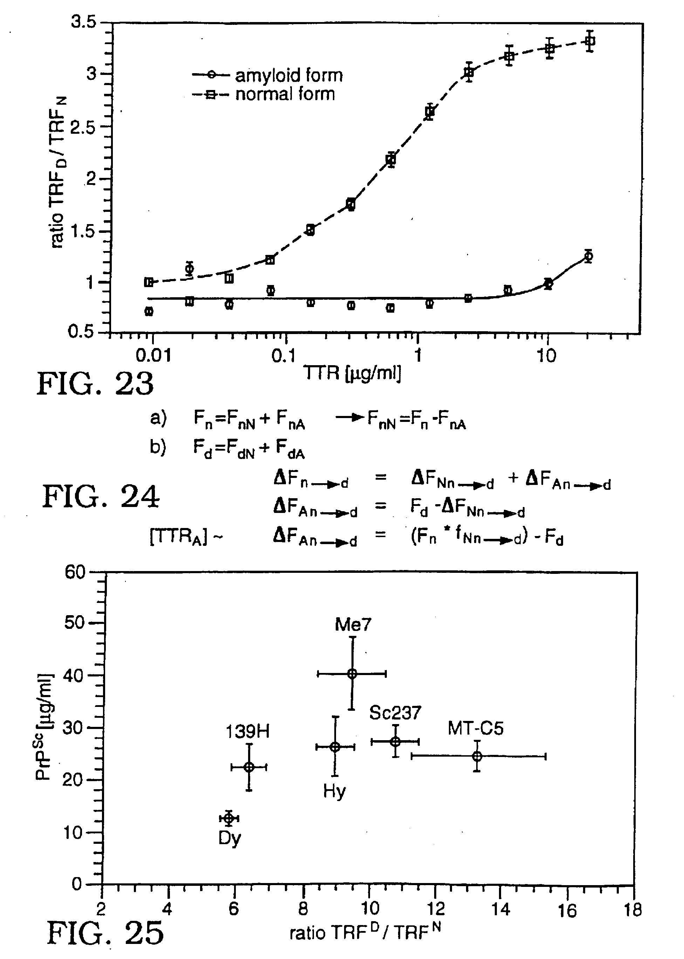

Figure 24: Modified formula for calculating the amount of TTR in amyloid conformation from the data obtained by direct assay with anti-TTR polyclonal antibody. The changes from general equation reflect the reversed ratio of denatured and native states for normal and amyloid forms of TTR. The difference between the fluorescence of denatured state of the sample and that expected for transition from native normal protein to denatured state is proportionate to the amount of TTR in amyloid conformation. $F_n$ - total signal of native conformation; $F_{nN}$ and $F_{nA}$ - the signals of native normal and amyloid conformations, respectively; $F_d$ - total signal of TTR in denatured state; $F_{dN}$ and $F_{dA}$ are the signals of denatured normal or amyloid states of TTR; $\Delta F_{n \to d}$ - the total increase of the signal in the transition from native to denatured states; $\Delta F_{Nn \to d}$ - increase in the signal of normal conformation in the transition from native to denatured state; $\Delta F_{An \to d}$ - change in the signal of amyloid conformation in the transition from native to denatured state; $f_{Nn \to d}$ - correlation factor for the transition from native to denatured state of normal TTR;

Figure 25 is a graph of the "prion index" (which is the ratio of antibody binding to denatured vs. native PrP protein) vs. the concentration of PrP$^{Sc}$ in μg/ml . The results shown represent the average ± SEM obtained from three different brains of LVG/LAK Syrian hamsters infected with different prion strains.

## DETAILED DESCRIPTION OF THE PREFERRED EMBODIMENTS

Before the present assays and methods are disclosed and described, it is to be understood that this invention is not limited to particular antibodies, proteins, labels, assays or method as such may, of course vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All publications mentioned herein are incorporated herein by reference to disclose and describe the methods and/or materials in connection with which the publications are cited.

The publications discussed herein are provided solely for the disclosure prior to the filing date of the present application. Nothing herein is to be construed as an admission that the present invention is not entitled to antedate such publication by virtue of prior invention. Further, the dates of publication provided are subject to change if it is found that the actual date of publication is different from that provided here.

## DEFINITIONS

The terms "protein" as used herein is intended to encompass any amino acid sequence and include modified sequences such as glycoproteins. The term includes naturally occurring proteins and peptides as well as those which are recombinantly or synthetically synthesized. As used in connection with the present invention the term "protein" is specifically intended to cover naturally occurring proteins which occur in at least two different conformations wherein both conformations have the same or substantially the same amino acid sequence but have different three dimensional structures. The two conformations of the protein include at least one conformation which is not related to a disease state and at least one conformation which is related to a disease state -- pathogenic. A specific and preferred example of a protein as used in connection with this disclosure is a PrP protein which includes the non-disease form referred to as the PrP$^c$ form and the disease related form referred as the PrP$^{Sc}$. Although a prion protein or the PrP$^{Sc}$ form of a PrP protein is infectious and pathogenic, the disease conformation of other proteins is not infectious although it is pathogenic. As used herein, the term pathogenic may mean that the protein actually causes the disease or it may simply mean that the protein is associated with the disease and therefore is present when the disease is present. Thus, a pathogenic protein as used in connection with this disclosure is not necessarily a protein which is the specific causative agent of a disease.

The terms "treating", "treatment" and the like are used interchangeably here to describe a process whereby a sample or portion thereof and specifically proteins in the sample are physically and/or chemically manipulated so that proteins in the sample in a disease related conformation are caused to changed to a different conformation with a higher binding affinity with a binding partner such as an antibody. Treated proteins are also referred to as denatured or partial denatured proteins or proteins in a relaxed conformation which conformation increases the binding affinity of the protein to a binding partner such as an antibody. Treating includes subjecting the sample to heat, pressure and/or chemicals. In a preferred embodiment, samples containing PrP$^{Sc}$ (which is the disease-related conformation comprising β-sheet structural configurations) are treated so that the protein assumes a different conformation (e.g., comprising an α-helical configuration and/or a random coil configuration) having four times or more greater antibody binding affinity.

The terms "PrP protein", "PrP" and like are used interchangeably herein and shall mean both the infectious particle form PrP$^{Sc}$ known to cause diseases (spongiform encephalopathies) in humans and animals and the noninfectious form PrP$^c$ which, under appropriate conditions is converted to the infectious PrP$^{Sc}$ form.

The terms "prion", "prion protein" and "PrP$^{Sc}$ protein" and the like we used interchangeably herein to refer to the infectious PrP$^{Sc}$ form of PrP, and is a contraction of the words "protein" and "infection." Particles are comprised largely, if not exclusively, of PrP$^{Sc}$ molecules encoded by a PrP gene. Prions are distinct from bacteria, viruses and viroids. Known prions infect animals to cause scrapie, a transmissible, degenerative disease of the nervous system of sheep and goats, as well as bovine spongiform encephalopathy (BSE), or "mad cow disease", and feline spongiform encephalopathy of cats. Four prion diseases known to affect humans are (1) kuru, (2) Creutzfeldt-Jakob Disease (CJD), (3) Gerstmann-Straussler-Scheinker Disease (GSS), and (4) fatal familial insomnia (FFI). As used herein "prion" includes all forms of prions causing all or any of these diseases or others in any animals used - and in particular in humans and domesticated farm animals.

The term "PrP gene" is used herein to describe genetic material which expresses proteins including known polymorphisms and pathogenic mutations. The term "PrP gene" refers generally to any gene of any species which encodes any form of a prion protein. Some commonly known PrP sequences are described in Gabriel et al., Proc. Natl. Acad. Sci. USA 89:9097-9101 (1992), and U.S. Patent 5,565,186 and WO97/04814, incorporated herein by reference to disclose and describe such sequences. The PrP gene can be from any animal, including the "host" and "test" animals described herein and any and all polymorphisms and mutations thereof, it being recognized that the terms include other such PrP genes that are yet to be discovered. The protein expressed by such a gene can assume either a PrP$^c$ (non-disease) or PrP$^{Sc}$ (disease) form.

The term "antibody" stands for an immunoglobulin protein which is capable of binding an antigen. Antibody as used herein is meant to include the entire antibody as well as any antibody fragments (e.g. F(ab)', Fab, Fv) capable of binding the epitope, antigen or antigenic fragment of interest. Preferred antibodies for assays of the invention are immunoreactive or immunospecific for and therefore specifically and selectively bind to a protein of interest e.g., an A4β amyloid protein or a PrP protein. Antibodies which are immunoreactive and immunospecific for both the native non-disease form and the treated disease form but not for the untreated disease form, (e.g., for both native PrP$^c$ and treated PrP$^{Sc}$ but not native PrP$^{Sc}$) are preferred. Antibodies for PrP are preferably immunospecific - e.g., not substantially cross-reactive with related materials. Some specific antibodies which can be used in connection with the invention are disclosed in published PCT application WO 97/10505 which is incorporated herein by reference to disclose and describe antibodies. This published PCT application corresponds to USSN 08/713,939 also incorporated herein by reference. Antibodies disclosed in the PCT application which selectively bind PrP$^{Sc}$ should not be used in the present invention. The term "antibody" encompasses all types of antibodies, e.g. polyclonal, monoclonal, and those produced by the phage display methodology. Particularly preferred antibodies of the invention are antibodies which have a relatively high degree of affinity for both native PrP$^c$ and treated PrP$^{Sc}$ but a relatively low degree of or substantially no binding affinity for PrP$^{Sc}$. More specifically, antibodies of the invention preferably have four times or more, more preferably fifteen times or more, and still more preferably 30 times or more binding affinity for both native PrP$^c$ and denatured PrP$^{Sc}$ as compared with the binding affinity for native PrP$^{Sc}$.

"Purified antibody" refers to that which is sufficiently free of other proteins, carbohydrates, and lipids with which it is naturally associated. Such an antibody "preferentially binds" to a treated or denatured disease conformation of a protein such as the β-sheet conformation of A4β or PrP$^{Sc}$ protein (or an antigenic fragment thereof), and does not substantially recognize or bind to other antigenically unrelated molecules. A purified antibody of the invention is preferably immunoreactive with and immunospecific for a specific species and more preferably immunospecific for native PrP$^c$ and for treated or denatured forms of PrP$^c$ and PrP$^{Sc}$ but not for native or untreated PrP$^{Sc}$.

"Antigenic fragment" of a protein (e.g., a PrP protein) is meant a portion of such a protein which is capable of binding an antibody.

By "binds specifically" is meant high avidity and/or high affinity binding of an antibody to a specific polypeptide e.g., epitope of a protein, e.g., a PrP$^c$ or A4β protein. Antibody binding to its epitope on this specific polypeptide is preferably stronger than binding of the same antibody to any other epitope, particularly those which may be present in molecules in association with, or in the same sample, as the specific polypeptide of interest e.g., binds more strongly to epitope fragments of a protein such as PrP$^{Sc}$ so that by adjusting binding conditions the antibody binds almost exclusively to an epitope site or fragments of a desired protein such as an epitope fragment exposed by treatment of PrP$^{Sc}$ and not exposed on native untreated PrP$^{Sc}$.

By "detectably labeled antibody", "detectably labeled anti-PrP" or "detectably labeled anti-PrP fragment" is meant an antibody (or antibody fragment which retains binding specificity), having an attached detectable label. The detectable label is normally attached by chemical conjugation, but where the label is a polypeptide, it could alternatively be attached by genetic engineering techniques. Methods for production of detectably labeled proteins are well known in the art. Detectable labels known in the art, but normally are radioisotopes, fluorophores, paramagnetic labels, enzymes (e.g., horseradish peroxidase), or other moieties or compounds which either emit a detectable signal (e.g., radioactivity, fluorescence, color) or emit a detectable signal after exposure of the label to its substrate. Various detectable label/substrate pairs (e.g., horseradish peroxidase/diaminobenzidine, avidin/streptavidin, luciferase/luciferin), methods for labeling antibodies, and methods for using labeled antibodies are well known in the art (see, for example, Harlow and Lane, eds. (Antibodies: A Laboratory Manual (1988) Cold Spring Harbor Laboratory Press, Cold Spring Harbor, NY)). Europium is a particularly preferred label.

Abbreviations used herein include:

CNS for central nervous system;
BSE for bovine spongiform encephalopathy;
CJD for Creutzfeldt-Jacob Disease;
FFI for fatal familial insomnia;
GSS for Gerstamnn-Strassler-Scheinker Disease;
Hu for human;
HuPrP for human prion protein;
Mo for mouse;
MoPrP for mouse prion protein;
SHa for a Syrian hamster;
SHaPrP for a Syrian hamster prion protein;
Tg for transgenic;
Tg(SHaPrP) for a transgenic mouse containing the
PrP gene of a Syrian hamster;
Tg(HuPrP) for transgenic mice containing the complete human PrP gene;
Tg(ShePrP) for transgenic mice containing the complete sheep PrP gene;
Tg(BovPrP) for transgenic mice containing the complete cow PrP gene;
$PrP^{Sc}$ for the scrapie isoform of the prion protein;
$PrP^{c}$ for the cellular contained common, normal isoform of the prion protein;
$MoPrP^{Sc}$ for the scrapie isoform of the mouse prion protein;
MHu2M for a chimeric mouse/human PrP gene wherein a region of the mouse PrP gene is replaced by a corresponding human sequence which differs from mouse PrP at 9 codons;
Tg(MHu2M) mice are transgenic mice of the invention which include the chimeric MHu2M gene;
$MHu2MPrP^{Sc}$ for the scrapie isoform of the chimeric human/mouse PrP gene;
$PrP^{CJD}$ for the CJD isoform of a PrP protein;
$Pmp^{0/0}$ for ablation of both alleles of an endogenous prion protein gene, e.g., the MoPrP gene;
$Tg(SHaPrP^{+/0})81/Prnp^{0/0}$ for a particular line (81) of transgenic mice expressing SHaPrP, +/0 indicates heterozygous;
$Tg(HuPrP)/Prnp^{\%}$ for a hybrid mouse obtained by crossing a mouse with a human prion protein gene (HuPrP with a mouse with both alleles of the endogenous prion protein gene disrupted;
$Tg(MHu2M)/Prnp^{0/0}$ for a hybrid mouse obtained by crossing a mouse with a chimeric prion protein gene (MHu2M) with a mouse with both alleles of the endogenous prion protein gene disrupted;
TTR for transthyretin;
FVB for a standard inbred strain of mice often used in the production of transgenic mice since eggs of FVB mice are relatively large and tolerate microinjection of exogenous DNA relatively well;
$[PrP_{\rho}]$ - concentration of prion protein in β-sheet conformation;
$[\beta A4_{\beta}]$ - concentration of βA4 in β-sheet conformation;
[DRC] - concentration of a disease related conformation of a protein.

GENERAL ASPECTS OF THE INVENTION

The assay method comprises providing a sample suspected of containing a protein which assumes a first conformation and a second disease related conformation and is capable of detecting a disease conformation of the protein when present in a very low concentration relative to the concentration of the non-disease conformation. The sample is divided into a first portion and a second portion. The first portion is preferably bound to the surface of the solid support and thereafter brought into contact with a labeled antibody. The antibody is of a type which binds to the protein in its first configuration with a higher (four times or more) degree of affinity than it binds to the protein in its second disease related conformation. The second portion of the sample is then treated in a manner which causes any protein in the second, disease related conformation to assume a different conformation which conformation has a higher degree of affinity (four times or more higher) for the labeled antibody as compared with the affinity for the protein in the untreated second disease related conformation. The treated second portion is then, preferably, bound to the surface of the solid support. The treated protein bound to the support is then contacted with a labeled antibody under conditions which allow the antibody to bind to proteins in the first configuration or proteins in the assumed different configuration.

After the labeled antibodies have been provided with sufficient time, temperature and chemical conditions (e. g., pH) to bind to the appropriate proteins present in the respective portions the level of binding of the labeled antibody to protein in each portion is determined. A highly sensitive assay is used such as an assay involving time-resolved, dissociation-enhanced fluorescence making it possible to detect concentrations in an amount in the range of about 1 x $10^3$ particles per ml or less. A high degree of sensitivity is required because in most samples the concentration of protein in the disease conformation will be very low in comparison to the concentration of the protein in the non-disease conformation, e.g., 3 orders of magnitude or more different. For example, the non-disease conformation of the protein might be present in an amount of about 1 x $10^8$ particles/ml while the disease conformation of the protein is only present in an amount of 1 x $10^4$ particles/ml. Thus, any increase in signal noted due to treating the disease conformation of the protein will be very small relative to the signal being obtained from the protein in the non-disease conformation.

After the level of binding for both portions of sample is obtained, the levels are compared. For example, the level of binding of labeled antibody to a protein in the first portion is subtracted from the level of binding of antibody to a protein in the second portion. The difference between the two reflects the amount of protein present in the original sample which was in the second, disease related conformation -- after adjusting for differences caused (if any) by increasing the binding affinity of protein in the first portion.

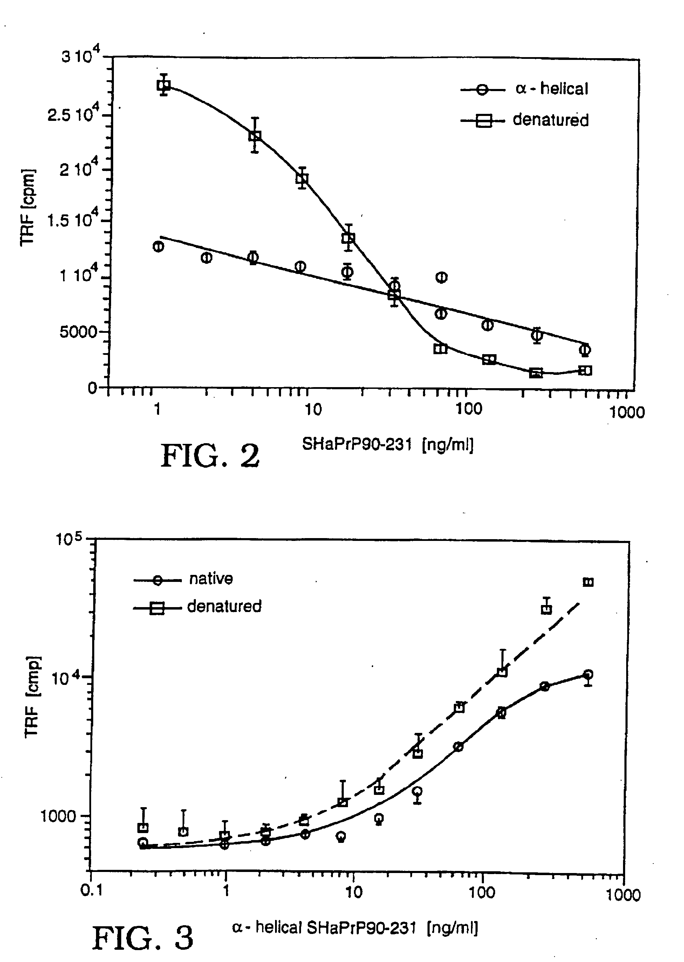

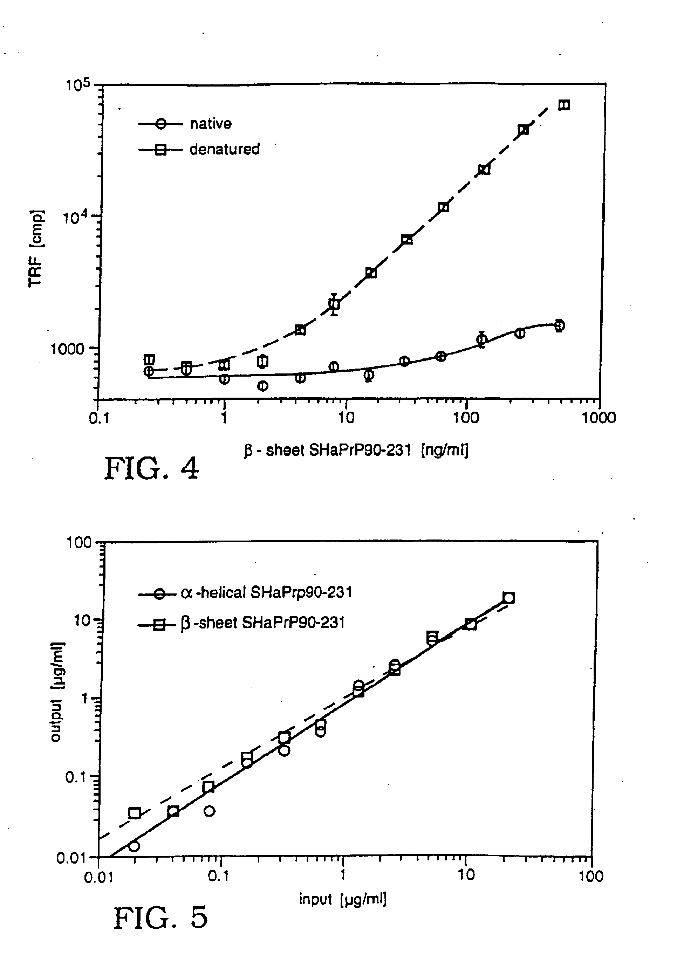

More specifically, with some proteins there may be some differences due to the effect of the treatment on the proteins which are in the native non-diseased conformation -- e.g., more epitopes of the protein in disease related conformation are exposed by treatment. This differential should be accounted for in drawing conclusions with respect to whether the original sample included proteins in the second, disease related conformation. Accounting for this effect is shown within Figures 3 and 4. In Fig. 3 there is shown a comparison of antibody binding to an untreated sample which contains only native protein in its non-disease configuration with the same native protein after treatment. As shown within Fig. 3 there is some difference between the results obtained with the treated protein showing a stronger signal in that the treatment increased the binding affinity of the protein. However, Fig. 4 shows the same results when the original sample included proteins which were in the second, disease related conformation. The native proteins which are not treated provide a very weak signal. However, the treated proteins provide a very strong signal. The large differential between the treated and the untreated samples is a clear indication that the original sample included proteins with the second, disease related conformation in that these proteins do not bind to antibodies or bind to antibodies with a very low degree of binding affinity. However, after treatment these proteins bind to the antibodies as well or nearly as well or better than the proteins in the non-disease conformation which were treated.

The assay can be used to test for the presence of the disease conformation of a given protein within any type of sample. Some of the most typical samples to be tested include pharmaceuticals which include components which are derived from living mammals or use materials derived from living mammals in their processing. It would also be desireable to test organs for transplantation and food items such as beef which was suspected of containing infectious prions. The invention could be used for testing for the presence of the disease conformation of one or more types of proteins such as infectious PrP$^{Sc}$ in pharmaceuticals, cosmetics, biopsy or autopsy tissue, brain, spinal cord, peripheral nerve, muscle, cerebrospinal fluid, blood and blood components, lymph nodes, and in animal or human-derived cultures infected or potentially infected by disease forms of proteins such as prions.

TREATING OR DENATURATION

As indicated above, "treating" can include exposing the proteins to any physical and/or chemical means which causes the protein which is originally present in a tightened, disease related conformation to assume a more relaxed conformation which has a higher degree of binding affinity for any binding partner such as antibodies. In general, the treatment or denaturing as it is sometimes referred to herein involves subjecting the protein to some means which causes epitopes on the protein which were not previously exposed to become exposed so that an antibody or other binding partner can bind to the newly exposed epitope. The methods of treatment which can be used include: (1) physical, such as hydrostatic pressure or temperature, (2) chemical, such as acidic or alkaline pH, chaotropic salts, denaturing detergents, and proteinases such as Proteinase K and (3) combinations of above.

The treatment time will vary depending on the treatment used but should be carried out for sufficient time to expose new binding sites but not so long as to completely denature the protein.

PRETREATING OR PROTEIN REMOVAL/DESTRUCTION

Before carrying out treatment or antibody testing of either portion of the sample it may be desirable to subject the sample to a pretreatment. The pretreatment is carried out in order to destroy or remove the non-disease form of the portein present within the sample. Examples of pretreatment methodology include producing a column which includes antibodies bound to support surfaces which antibodies bind to the non-disease conformation of the protein thereby removing as much of the non-disease conformation of the proteins possible. Alternatively the sample can be subjected to physical treatment such as long term hydrostatic pressure of temperature alone or in combination with chemicals such as acids or alkalines as indicated above in the "treatment" phase but carried out for a longer period of time and in a manner so as to destroy proteins present in the sample which proteins are in the non-disease conformation. In some instances proteins in the non-disease and disease conformation will be destroyed. However, a higher relative percentage of the proteins in the non-disease conformation will be destroyed because these proteins are initially in a looser conformation which is more vulnerable to destruction. Thus, the pretreatment methodology results in a sample which includes a relatively lower concentration of the non-disease conformation of the protein relative to the concentration of the disease conformation of the protein. This increases the sensitivity of the assay making it possible to detect lower concentrations of the disease conformation of the protein. Removal of proteins is preferred over destruction of such in that destruction will decrease sensitivity if the disease conformation is destroyed. A particularly useful pretreatment method is disclosed in our patent application attorney docket 6510/098001 filed on the same date as the present application entitled "Process for Concentrating Protein with Disease-Related Conformation".

BINDING PROTEINS TO SUPPORT SURFACES

The method of chemical or affinity coupling of PrP protein to the plastic support are generally described in available literature and may vary. The antibodies used in the diagnostic assay are polyclonal, monoclonal or recombinant Fab and need to be species specific with preferential binding to the native $PrP^c$ or denatured form of $PrP^{Sc}$ with preferably at least 4-fold lower reactivity with infectious $PrP^{Sc}$, assuming the same amount of the antigen.

USING THE ASSAY TO DETECT PRIONS

One aspect of the invention is a two step process to diagnose prion disease by quantitatively measuring native infectious form of $PrP^{Sc}$ protein in sample material or in the brains of susceptible animals inoculated with such material. The sample is divided into two aliquots. The first aliquot is crosslinked to the solid plastic support in native conformation through a chemical activation step under the non-denaturing conditions, i.e., no treating. The second portion of the sample is first treated and then crosslinked to the plastic support. Both portions of the sample material react in situ with the labeled antibodies that preferentially recognize native $PrP^C$ or treated $PrP^{Sc}$ of the given animal species. The amount of the antibody bound to treated or native conformations of PrP protein is recorded by the signal of the IgG label. The excess of the signal obtained with the denatured sample over that expected from an increase in the signal obtained with the native $\alpha$-helical conformation of $PrP^c$ (i.e., the increase over the adjusted amount)is the measure of the amount of infectious $\beta$-sheet structured $PrP^{Sc}$ in the original sample. The formula developed for calculation of $PrP^{Sc}$ content is shown in formulae provided here and exemplified in Example 11.

The diagnosis of prion disease is established by three procedures: (1) measurement of treated sample alone and by detecting the increase in the total PrP amount in the examined sample above the background levels of $PrP^c$ obtained from normal controls; (2) calculation of the ratio between denatured versus native signal for a given antibodies - for example values higher than 2.2 for Europium-labeled 3F4 IgG indicates presence of $PrP^{Sc}$ preferably using time-resolved, dissociation-enhanced fluorescence; (3) evaluation of the excess of the denatured sample signal over that expected from increase in the signal for $\alpha$-helical conformation of $PrP^c$ as a measure of the amount of infectious $\beta$-sheet structured $PrP^{Sc}$ in the original sample. Preferably prior to step (1) the method uses a pre-treatment step whereby $PrP^c$ is removed or destroyed in relative amounts greater than that of $PrP^{Sc}$.

In a scrapie infected Syrian hamster brain, the concentration of $PrP^{Sc}$ is 5-10 times higher than $PrP^c$ when the animals become ill. At this time, the prion titer in their brains is $10^7 - 10^8$ $ID_{50}$ units/ml of 10% homogenate. The highly quantitative system that we have developed allows us to subtract the $PrP^c$ signal. The subtraction can be readily carried out when the concentration of $PrP^{Sc}$ is greater than $PrP^c$. However, the subtraction becomes difficult when the concentration of $PrP^{Sc}$ is much less than $PrP^c$.

To address the foregoing problem, the present assay utilizes the principle of affinity between different conformations of antigen and antibody. To measure the concentration of $PrP^{Sc}$ when it is much less than $PrP^c$, the detection system has to have extreme sensitivity and a linear range of at least $10^4$. The assay described herein can readily detect $PrP^{Sc}$ at a concentration of (approximately) 50 pg/ml using Europium-labeled IgG. Assuming $10^5 - 10^6$ $PrP^{Sc}$ molecules per $ID_{50}$ unit the present assay can readily detect $5 \times 10^2 - 5 \times 10^3$ $ID_{50}$ units per ml.

The assay can detect $PrP^{Sc}$ in mixtures (by direct method) where the concentration of $PrP^{Sc}$ is less than 1% of the concentration of $PrP^c$. Additional sensitivity can be achieved by immunoprecipitation, using a sandwich format for a solid state assay, differential centrifugation with detergent extraction to remove $PrP^c$, the indirect transgenic animal method or combinations of these methods. A conservative estimate is that such procedures should allow measurement of between 5 and 50 $ID_{50}$ units per ml or less conservatively to measure between 0.1 and 0.01 $ID_{50}$ units per ml. Such measurements would provide a rapid, "positive" means of establishing biological sterility which is the "absence" of infectivity.

METHOD OF DRUG IDENTIFICATION

The assays described above which can identify the presence of a disease related conformation of a protein can be applied to identifying compounds which could then be used as drugs in treating or preventing diseases associated with the disease conformation of the protein. For example, compositions which are known to include disease related conformations of a protein can be used to inoculate a transgenic mouse of the type disclosed and described in U.S. Patent 5,565,186 and PCT publication WO97/04814. The inoculated mouse can then be treated with a compound which is believed to prevent the infection. After providing a sufficient period of time for incubation, the mouse brain is extracted and tested using the assay described above to determine if the treatment compound was effective in preventing infection. The methodology could also be applied to situations where the infection had already taken place in order to determine if a particular compound could stabilize the infection i.e., prevent further formation of the disease conformation of the protein.

The assay of the present invention can be used in combination with the methodology disclosed within earlier filed USSN 08/556,823, filed November 2, 1995, incorporated herein by reference. This application discloses compounds which can be brought into contact with proteins in a non-disease conformation in order to convert those compounds to a disease conformation. The methodology is useful in that it allows one to screen compounds for their ability to prevent the formation of the disease related conformation of the protein. The assay of the present invention makes it possible to accurately measure the effect of any test compound on preventing the formation of the disease related conformation.

Based on the above it can be seen that the invention includes a method of screening compounds which affect the conformational shape of any protein such as a PrP protein which has a first non-disease related conformation (e. g., PrP$^C$) and a second disease related conformation (e.g., PrP$^{Sc}$). The method involves first providing a sample having the protein present in the first, non-disease conformation. The sample is then brought into contact with a test compound which is being screened for its therapeutic utility. After adding the test compound the sample is then brought into contact with a compound or group of compounds which induce the protein in the first, non-disease conformation to convert to the second, disease-conformation. After allowing for a sufficient period of time the sample is assayed using the present invention. The assay of the present invention will make it possible to accurately determine how the test compound affected the conversion of the protein from the non-disease conformation to the disease conformation. It will be apparent to those skilled in the art reading this disclosure that the test compound can be added at different points in time relative to the addition of the compound which affects the conformational change. Thereby, the methodology can be used to determine the ability of a test compound to stabilize further changes from the non-disease conformation to the disease conformation and/or determine the ability of the test compound to prevent the initiation of any conversion from the non-disease conformation to the disease conformation. Although USSN 08/556,823 discloses a means for converting a non-disease conformation to a disease conformation, other means for obtaining the same result will become apparent to those skilled in the art upon reading the present application and reviewing the state of the art in connection with such. Accordingly, the present invention is intended to encompass any physical, chemical or biological means which would be utilized to convert a protein within a first, non-disease conformation to a second, disease conformation and applying the assay described here. Further, the present invention is intended to encompass therapeutic compounds which are obtained as a result of carrying out the screening method of the invention, i.e., compounds produced by the method of the invention.

ANTIBODIES

Method of generating antibodies are generally known to those skilled in the art. In that the disease form is often in a tighter configuration than the non-disease form, with less epitopes exposed, one can readily generate antibodies which bind only to the non-disease form of the protein or the treated disease form. For example, antibodies detecting treated forms of PrP$^{Sc}$ protein and PrP$^c$ protein may be generated by immunizing rabbits or mice with $\alpha$-helical conformations of recombinant PrP, native PrP$^c$ from animal brains, synthetic peptides in $\alpha$-helical or random coil conformations, or against denatured PrP$^{Sc}$ or PrP 27-30. Only antibodies with affinity at least 4 fold higher for PrP$^c$ (or denatured conformation of PrP$^{Sc}$ of the same species) as compared to their affinity for PrP$^{Sc}$ should be selected. The method of antibody generation, purification, labeling and detection may vary.

The IgG or Fab's may be purified from different sources by affinity HPLC using protein A column and Size exclusion HPLC. The purified antibodies may be labeled with Europium and detected by time resolved fluorescence.

The antibody binding to different conformations of PrP protein may be measured by time-resolved, dissociation-enhanced fluorescence. However, the system of detection of PrP-bound IgG on solid support in situ or in solution may vary. Further, it is possible to use direct or indirect immunological methods including direct radiolabels, fluorescence, luminescence, avidin-biotin amplification, or enzyme-linked assays with color or luminescent substrates.

An antibody which can be used in the invention is disclosed in US 4,806,627, issued February 21, 1989, disclosing monoclonal antibody 263K 3F4, produced by cell line ATCC HB9222 deposited on October 8, 1986, which is incorporated herein by reference. The cell line producing the antibody can be obtained from the American Type Culture Collection, 12301 Parklawn Drive, Rockville, MD 20852.

In general, scrapie infection fails to produce an immune response, with host organisms being tolerant to $PrP^{Sc}$ from the same species. Antibodies which bind to either $PrP^c$ or $PrP^{Sc}$ are disclosed in WO97/10505, published March 20, 1997. Any antibody binding to $PrP^c$ and not to $PrP^{Sc}$ can be used, and those skilled in the art can generate such using known procedures, e.g., see methods of producing page display antibody libraries in US 5,223,409. Polyclonal anti-PrP antibodies have though been raised in rabbits following immunization with large amounts of formic acid or SDS-denatured SHaPrP 27-30 [Bendheim, Barry et al. (1984) Nature 310:418-421; Bode, Pocchiari et al. (1985) J Gen Virol 66:2471-2478; Safar, Ceroni et al. (1990) Neurology 40:513-517]. Similarly, a handful of anti-PrP monoclonal antibodies against PrP 27-30 have been produced in mice [Barry and Prusiner (1986) J Infect Dis 154:518-521; Kascsak, Rubenstein et al. (1987) J Virol 61:3688-3693]. These antibodies were generated against formic acid- or SDS-denatured PrP 27-30 and are able to recognize native $PrP^c$ and treated or denatured $PrP^{Sc}$ from both SHa and humans equally well, but do not bind to MoPrP. Not surprisingly, the epitopes of these antibodies were mapped to regions of the sequence containing amino acid differences between SHa- and MoPrP [Rogers, Yehiely et al. (1993) Proc Natl Acad Sci USA 90:3182-3186].

It is not entirely clear why many antibodies of the type described in the above cited publications will bind to $PrP^c$ and treated or denatured $PrP^{Sc}$ but not to native $PrP^{Sc}$. Without being bound to any particular theory it is suggested that such may take place because epitopes which are exposed when the protein is in the $PrP^c$ conformation are unexposed or partially hidden in the $PrP^{Sc}$ configuration - where the protein is relatively insoluble and more compactly folded together.

For purposes of the invention an indication that no binding occurs means that the equilibrium or affinity constant $K_a$ is $10^6$ l/mole or less. Further, binding will be recognized as existing when the $K_a$ is at $10^7$ l/mole or greater, preferably $10^8$ l/mole or greater. The binding affinity of $10^7$ l/mole or more may be due to (1) a single monoclonal antibody (i.e., large numbers of one kind of antibodies) or (2) a plurality of different monoclonal antibodies (e.g., large numbers of each of five different monoclonal antibodies) or (3) large numbers of polyclonal antibodies. It is also possible to use combinations of (1) - (3). Selected preferred antibodies will bind at least 4-fold more avidly to the treated or denatured $PrP^{Sc}$ forms of the protein when compared with their binding to the native conformation of $PrP^{Sc}$. The four fold differential in binding affinity may be accomplished by using several different antibodies as per (1) - (3) above and as such some of the antibodies in a mixture could have less than a four fold difference.

A variety of different types of assays of the invention may be used with one or more different antibodies. Those skill in the art will recognize that antibodies may be labeled with known labels and used with currently available robotics, sandwich assays, electronic detectors, flow cytometry, and the like.

QUANTITATIVE CALCULATIONS

Using the methodology described above it is possible to calculate the difference between the amount of signal obtained from a sample which has not been treated and the signal obtained with a sample which has been treated. This difference represents (after adjusting for the effect of treatment on the non-disease conformation) the amount (concentration) of protein in disease conformation present in the original sample. After obtaining the difference the formula put forth below can be used to calculate the amount of protein in the disease conformation present in the original sample per unit of volume.

a) $F_n = F_{n\alpha} + F_{n\beta} \rightarrow F_{n\alpha} = F_n - F_{n\beta}$, $F_{n\beta} \sim$ background

b) $F_d = F_{d\alpha} + F_{d\beta}$

$$\Delta F_{n \rightarrow d} = \Delta F_{\alpha n \rightarrow d} + \Delta F_{\beta n \rightarrow d}$$

$$\Delta F_{\beta n \rightarrow d} = F_d - F_n - \Delta F_{\alpha n \rightarrow d}$$

$$[PrP_\beta] \text{ or } [DRC] \sim \Delta F_{\beta n\text{-}d} = F_d - (F_n * f_{\alpha n\text{-} \to d})$$

The definition of each of the above variables is provided below.

F - fluorescence signal (note that any detectable signal could be used);
$F_n$ - fluorescence signal of native conformation;
$F_{n\alpha}$ and $F_{n\beta}$ fluorescence signals of native $\alpha$-helical and $\beta$-sheet conformations, respectively;
$F_d$ - fluorescence signal of PrP in the treated or denatured state;
$F_{d\alpha}$ and $F_{d\beta}$ - are the signals of denatured $\alpha$-helical of $\beta$-sheet states of PrP;
$\Delta F_{n \to d}$ - increase of the fluorescence signal in the transition from native to denatured state;
$\Delta F_{\alpha n \to d}$ - increase in the fluorescence signal of $\alpha$-helical conformation in the transition from native to denatured state;
$\Delta F_{\beta n \to d}$ - increase in the signal of $\beta$-sheet conformation in the transition from native to denatured state;
$f_{\alpha n \to d}$ - correlation factor for the transition from native to denatured state of $\alpha$-helical PrP;
$[PrP_\beta]$ - concentration of prion protein in $\beta$-sheet conformation.
[DRC] - concentration of any protein in disease related conformation.

The formula provided above is used to specifically calculate the concentration of prion protein in the $\beta$-sheet conformation. However, the same formulae can be used to calculate the concentration of any protein i.e., the concentration of any constricted, disease conformation of a protein such a $[\beta A4_\beta]$. More generally, [DRC] represents the concentration of the disease related conformation of a protein.

To provide a specific example, the above definitions have been provided specifically with respect to PrP proteins which proteins include at least one relaxed, non-disease conformation (PrP[c]) which includes an $\alpha$-helical configuration and at least one constricted, disease related conformation (PrP[Sc]) which includes a $\beta$-sheet configuration. The formulae are used to calculate the concentration of the disease related conformation of the protein present in the sample. Per the specific formulae and definitions provided above the formulae are used to calculate the concentration of prion proteins which include the $\beta$-sheet configuration (see Example 11).

The signal used in calculating the above formula is a fluorescence signal. However, any detectable signal can be used. The total signal is represented by $F_n$ which is a combination of the signal received from the disease and the non-disease related conformations. This is a signal which would be calculated from portion No. 1 which is not treated per the assay described above. The variable $F_d$ is the signal which is obtained by treating portion No. 2 of the sample. This signal is a combination of the signal received from treated protein in the non-disease conformation plus treated protein in the disease conformation.

It has been recognized that there is a difference in signal obtained by treating a sample which includes no disease related conformation of the protein. The difference should be accounted for to obtain an accurate reading. The difference in signal obtained between the native sample and the treated sample is, of course, a combination of the difference in signal obtained by treating the disease related conformation and the non-disease conformation. The increase in the signal obtained by treating the disease conformation, i.e., the difference between the signal of the untreated disease conformation and the signal received from the treated disease conformation can be calculated by subtracting the signal received from treating the entire sample from the signal received from calculating the increase in signal obtained from the untreated non-disease conformation and the treated non-disease conformation. Using these equations it is possible to produce the final equation which provides the concentration of protein in the disease conformation present in the original sample (see Example 11).

DIFFERENTIATING (TYPING) OF PROTEIN STRAINS

Different animals, including humans may become infected with different strains of pathogenic proteins. A "mutation table" is provided here to list some of the different mutations associated with different strains of prion infections. At times it may be important which specific strain has infected an individual in that such information may be useful in (1) providing a more precise diagnosis, (2) administering the appropriate treatment or (3) determining the source of the infection by matching the strain to a strain in a probable source of infection.

The particular strain of pathogenic protein causing an infection can be determined from two pieces of information which can be calculated using the present invention. Example 11 shows how the present invention can be used to determine the absolute amount, i.e., the concentration of prions in a sample of given size. Example 8 shows how to calculate the "prion index" which is the ratio of antibody binding to denatured:native PrP protein. A "protein index" for other proteins is the ratio of binding of any binding partner to the denature:native form of the protein. In general terms, the "prion index" is simply a numerical characterization of the affect the treatment has on the protein.

To determine the particular strain one must first calculate a standard for each strain. This is done by determining the affect (prion index) of a particular treatment on a known amount of a known strain. This can be plotted as shown in Figure 25. Once the standard is calculated, the strain of other samples can be readily determined -- standards are preferably calculated at a number of different concentrations for each strain. To determine the strain of a simple sample, one calculates the concentration of protein in the sample and the affect of treatment on that concentration. The results are matched to the standard (as in Figure 25) to determine the strain. Example 18 is a specific example of such as taken in combination with Figure 25. The same concentration of the same strain will be effected in the same way by a given treatment. However, the same concentration of different strains will be effected differently by the same treatment making it possible to determine the strain.

DISEASES ASSOCIATED WITH INSOLUBLE PROTEINS

Much of the disclosure and the specific examples provided herein relate to the use of the assay in connection with determining the presence of $PrP^{Sc}$ in the sample. However, as indicated above, the assay of the invention can be applied to determining the presence of any protein which assumes two different conformational shapes, one of which is associated with the disease. The following is a non-limiting list of diseases with associated insoluble proteins which assume two or more different conformations.

| Disease | Insoluble Proteins |
|---|---|
| Alzheimer's Disease | APP, $A\beta$ peptide, $\alpha$1-antichymotrypsin, tan, non-$A\beta$ component |
| Prion diseases, Creutzfeld Jakob disease, scrapie and bovine spongeform encephalopathy | $PrP^{Sc}$ |
| ALS | SOD and neurofilament |
| Pick's disease | Pick body |
| Parkinson's disease | Lewy body |
| Diabetes Type 1 | Amylin |
| Multiple myeloma-plasma cell dyscrasias | IgGL-chain |
| Familial amyloidotic polyneuropathy | Transthyretin |
| Medullary carcinoma of thyroid | Procalcitonin |
| Chronic renal failure | $\beta_2$-microglobulin |
| Congestive heart failure | Atrial natriuretic factor |
| Senile cardiac and systemic amyloidosis | Transthyretin |
| Chronic inflammation | Serum amyloid A |

(continued)

| Disease | Insoluble Proteins |
|---|---|
| Atherosclerosis | ApoA1 |
| Familial amyloidosis | Gelsolin |

It should be noted that the insoluble proteins listed above each include a number of variance or mutations which result in different strains which are all encompassed by the present. Known pathogenic mutations and polymorphisms in the PrP gene related to prion diseases are given below and the sequences of human, sheep and bovine are given in US 5,565,186, issued October 15, 1996.

| MUTATION TABLE | | | |
|---|---|---|---|
| **Pathogenic human mutations** | **Human Polymorphisms** | **Sheep Polymorphisms** | **Bovine Polymorphisms** |
| 2 octarepeat insert | Codon 129 Met/Val | Codon 171 Arg/Glu | 5 or 6 octarepeats |
| 4 octarepeat insert | Codon 219 Glu/Lys | Codon 136 Ala/Val | |
| 5 octarepeat insert | | | |
| 6 octarepeat insert | | | |
| 7 octarepeat insert | | | |
| 8 octarepeat insert | | | |
| 9 octarepeat insert | | | |
| Codon 102 Pro-Leu | | | |
| Codon 105 Pro-Leu | | | |
| Codon 117 Ala-Val | | | |
| Codon 145 Stop | | | |
| Codon 178 Asp-Asn | | | |
| Codon 180 Val-Ile | | | |
| Codon 198 Phe-Ser | | | |
| Codon 200 Glu-Lys | | | |
| Codon 210 Val-Ile | | | |
| Codon 217 Asn-Arg | | | |
| Codon 232 Met-Ala | | | |

It should also be noted that such proteins have two different 3-dimensional conformations with the same amino acid sequence. One conformation is associated with disease characteristics and is generally insoluble whereas the other conformation is not associated with disease characteristics and is soluble. The methodology of the present invention is not limited to the diseases, proteins and strains listed.

DETECTING THE β-SHEET FORM OF βA4

One aspect of the invention involves a two step process to diagnose Alzheimer's disease based on the presence of a constricted form of a protein (βA4 amyloidosis) by quantitatively measuring β-sheet form of βA4 protein in sample material, e.g., in the brain or body fluids. The sample is divided into two aliquots. The first aliquot is crosslinked to a solid plastic (long chain polymeric material) support in native conformation through a chemical activation step under the nondenaturing conditions. The second portion of the sample is first denatured and then crosslinked to the plastic support. Both portions of the sample material react in situ with the labeled antibodies that preferentially recognize soluble βA4 or denatured βA4 of the human or a given animal species. The amount of the antibody bound to denatured or native conformations of βA4 protein is recorded by the signal of the labeled secondary antibody. The excess of the signal obtained with the denatured sample compared to that expected change in the signal obtained with the native α-helical conformation of βA4 protein is the measure of the amount of β-sheet structured βA4 in the original sample. The formula developed for calculation of βA4 content is provided above in connection with the calculation of PrP$^{Sc}$ content.

The diagnosis of βA4 amyloidosis (Alzheimer's disease) is established by three procedures: (1) measurement of denatured sample alone and by detecting the increase in the total βA4 amount (concentration) in the examined sample above the background levels of soluble βA4 obtained from normal controls; (2) calculation of the ratio between denatured versus native signal for a given antibodies (protein index) - for example values higher than 2 for monoclonal antibody 6F3D and europium labeled secondary antibody; (3) evaluation of the change of the denatured sample signal over that expected change in the signal for α-helical conformation of βA4 as a measure of the amount of infectious β-sheet structured βA4 in the original sample. The formula developed for calculation of βA4 content is provided above. The particular strain of βA4 can also be determined using the same methodology described above to determine the strain of PrP$^{Sc}$ in a sample.

The invention provides a direct diagnostic method for detecting the presence pathogenic forms of βA4 protein in pharmaceuticals, biopsy or autopsy tissue, brain, spinal cord, peripheral nerves, muscle, cerebrospinal fluid, blood and blood components, lymph nodes, and in animal- or human-derived cultures expressing or potentially expressing βA4 protein. The invention also makes it possible to follow the α-helix-to-β-sheet conformational transition of βA4 protein, or its fragments of synthetic or recombinant origin, and to provide a method to screen compounds for their ability to stabilize the normal soluble conformation of βA4 protein and thus prevent conversion into pathogenic insoluble and β-sheet-structured βA4 protein.

Typical methods of sample denaturation include: (1) physical, such as hydrostatic pressure or temperature, (2) chemical, such as acidic or alkaline pH, chaotropic salts, or denaturing detergents, and (3) combination of above. Methods of chemical or affinity coupling of βA4 protein to a plastic support are described in available literature and may vary. Antibodies used in the diagnostic assay may be polyclonal, monoclonal or recombinant Fab and must be species specific with preferential binding to the soluble or denatured form of βA4 with preferably at least a 2-fold difference in reactivity between α-helical and β-sheet structured βA4, assuming the same amount of antigen.

Methods of sample attachment to the plastic support may vary and may be covalent or non-covalent as described in available literature. The sensitivity of the assay described in the examples may be increased by using high-affinity antibodies, sandwich formate, immunoprecipitation, or differential centrifugation. However, only the antibodies with an affinity at least a 2 fold for denatured and compared to the native β-sheet conformation of βA4 of the same species shall be used for the diagnostic assay. Methods of antibody generation, purification, labeling and detection may vary. The antibody binding to different conformations of βA4 protein was measured by time-resolved, dissociation-enhanced fluorescence. However, the system of detection of βA4-bound IgG on solid support in situ or in solution may vary and may use direct or indirect immunological methods including direct radiolabels, fluorescence, luminescence, avidin-biotin amplification, or enzyme-linked assays with color or luminescent substrates.

EXAMPLES

The following examples are put forth so as to provide those of ordinary skill in the art with a complete disclosure and description of how to make and use assays of the present invention, and are not intended to limit the scope of what the inventors regard as their invention, nor are they intended to represent or imply that the experiments below are all of or the only experiments performed. Efforts have been made to ensure accuracy with respect to numbers used (e.g. amounts, temperature, etc.) but some experimental errors and deviations should be accounted for. Unless indicated otherwise; parts are parts by weight, molecular weight is weight average molecular weight, temperature is in degrees centigrade, and pressure is at or near atmospheric.

**EXAMPLE 1**

EXPRESSION OF RECOMBINANT PRION PROTEINS

For the development and calibration of the diagnostic assays, recombinant Syrian hamster prion proteins of sequence 90-231 were refolded into α-helical or β-sheet conformations as described [Mehlhorn, Groth et al. (1996) Biochemistry 35:5528-5537]. PCR (Perkin-Elmer) was used to amplify the DNA corresponding to different portions of the Syrian hamster prion protein in order to ligate it into *E. coli* secretion vectors. Several 5' oligonucleotide primers were synthesized with an Mlu I restriction site within the C-terminal coding sequence of the STII signal peptide [Lee, Moseley et al. (1983) Infect Immun 42:264-268; Picken, Mazaitis et al. (1983) Infect Immun 42:269-275] and the initial amino acids of the appropriate PrP sequence. One 3' oligonucleotide primer matching the 3' end of PrP, a stop codon and a Bam HI restriction site was used with each of the 5' oligonucleotides. The PCR amplified products were purified, ligated into the vectors previously digested with MluI/Bam HI and transformed into DH5a. Clones containing the PrP insert were sequenced and transformed into the protease deficient expression strain 27C7 (ATCC# 55244).

Large scale expression was carried out as described previously for other proteins using a different medium [Carter, Kelley et al. (1992) Biotechnology 10:163-167]; 500 mL of an overnight culture grown in LB medium supplemented with ampicillin was inoculated into 7 L of fermentation medium in an aerated 10 L fermentor (Braun, model E10). Cells were grown at 37°C at a high agitation rate, and expression was induced by phosphate starvation. After 4 h, a 50% glucose solution was added at a rate of 1 mL/min; glucose levels were monitored using a glucose dipstick (Diastix, Miles Inc.). A pH of 7.4 was maintained throughout the run by the automated addition of 10% $H_2SO_4$ or 24% $NH_4OH$. The final volume was 10 L in which an $OD_{600}$ of $\geq$ 100 was achieved after 36 h. The *E. coli* was harvested by centrifugation at 10,000 x g for 30 min and the resulting paste was stored at -20°C.

For purification, 100 g of *E. coli* paste was resuspended in 1 L of 25 mM Tris·HCl, pH 8.0, 5 mM EDTA (buffer A). This was centrifuged at 10,000 x g for 20 min, and the supernatant containing soluble periplasmic proteins was discarded. The pellet was resuspended in 1 L of buffer A, passed through a cell disrupter twice (Microfluidics International, model MF 110), and centrifuged at 30,000 x g for 1 h, after which the supernatant was discarded and the pellet was washed once in buffer A and centrifuged again at 30,000 x g for 1 h. At this stage the pellet could be stored at -20°C prior to further separation. It was subsequently solubilized in 8M Gdn·HCl/25 mM Tris-HCl, pH 8.0/100 mM DTT (buffer B) and centrifuged at 14,000 x g for 20 min to remove the remaining insoluble matter. Aliquots of 6 mL of the supernatant containing $\sim$200 mg total protein were separated by size exclusion chromatography (SEC) using a 26 mm x 60 cm HiLoad Superdex 200 column (Pharmacia), eluting with 6M Gdn·HCl/12.5 mM Tris-HCl, pH 8.0/5mM DTT/ 1 mM EDTA (buffer C) at a flow rate of 2 mL/min. Fractions enriched for the recombinant prion protein as identified by SDS-PAGE were pooled and further purified by reversed phase high performance liquid chromatography (RP-HPLC) employing a 25 mm x 25 cm C-4 column (Vydac); Buffer 1: $H_2O$/0.1% TFA, Buffer 2: acetonitrile/0.09% TFA, flow rate 5 mL/min. The recombinant protein rPrP was found in fractions containing 40% acetonitrile. If the SEC eluate was stored at 4°C for several days prior to RP-HPLC, the recombinant protein was eluted in earlier fractions containing only 35% acetonitrile.

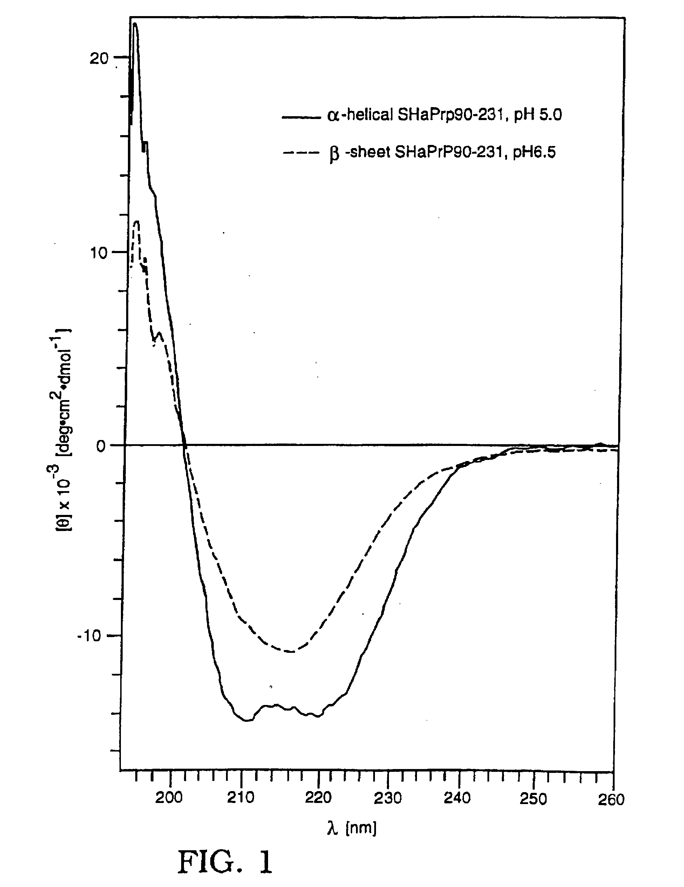

Samples of the reduced protein and the refolded oxidized form were concentrated using a Centricon column (Amicon) with a molecular weight cut-off of 10,000 Da. The buffer for the reduced protein was 10 mM MES, pH 6.5 whereas the oxidized form was concentrated in the refolding buffer described above. The conformations of refolded oxidized and reduced forms of SHaPrP90-231 protein were determined by circular dichroism (CD) spectroscopy (Fig. 1).

## EXAMPLE 2

### PURIFICATION OF HAMSTER PrP^C FROM NORMAL AND PrP^Sc FROM SCRAPIE INFECTED HAMSTER BRAINS

Both proteins produced per Example 1 were used as a standards for the prion assay and to establish the sensitivity and linearity range of the diagnostic method. The purified Syrian hamster brain PrP^c was used for the calibration of prion protein detection and correlated with results obtained on recombinant SHaPrP90-231 in α-helical, β-sheet, and denatured conformations. The PrP^c protein was purified as described with some minor modifications [Pan, Stahl et al. (1992) Protein Sci 1:1343-1352; Pan, Baldwin et al. (1993) Proc Natl Acad Sci USA 90:10962-10966]. Protein content was determined by amino acid analysis. The purity of PrP^c protein, as demonstrated on SDS PAGE followed by silver staining and Western, was $\geq$ 95%.

Standard Syrian hamster PrP^Sc was purified from a standard pool of scrapie strain Sc237 infected hamster brains as described with only minor modifications [Turk, Teplow et al. (1988) Eur J Biochem 176:21-30]. The infectivity of this standard, as determined by an incubation time assay on Syrian hamsters after intracerebral inoculation, was $10^{7.3}$ $ID_{50}$/ml and specific infectivity $10^{8.2}$ $ID_{50}$/mg of PrP^Sc protein. However, the specific infectivity may vary from lot to lot$\pm$ $10^{0.5}$ $ID_{50}$/mg. The protein content was determined by BCA assay using Bovine serum albumin as a standard. The preparation was considered homogeneous with one major band on SDS PAGE after silver staining and Western Blots.

## EXAMPLE 3

### SELECTION LABELING AND DETECTION METHOD OF ANTIBODIES USED IN THE ASSAY

The protocols and methods of antibody production and characterization are in general described elsewhere [Harlow and Lane (1988) supra: 726]. The data described in this and following examples were generated with immunoaffinity purified polyclonal antibody N12 and P3 [Safar, Ceroni et al. (1990) Neurology 40:513-517: Rogers, Serban et al. (1991)J Immunol 147:3568-3574], made against synthetic peptides corresponding sequence 90-145 (N12) and 222-231 (P3) of Syrian Hamster PrP [Barry, Vincent et al. (1988) J Immunol 140:1188-1193]; JS2 against denatured Syrian Hamster PrP 27-30 [Safar, Ceroni et al. (1990) Neurology 40:513-517]. The development and characteristics of monoclonal antibody 3F4 used in the assay are described elsewhere [Kascsak, Rubenstein et al. (1987) J Virol 61: 3688-3693] and are described in US 4,806,627, all of which are incorporated by reference to disclose and describe antibodies which can be used with the invention and methods of making those and related antibodies. The recombinant Fab recognizing denatured forms ofprion protein were developed most recently [Williamson, Peretz et al. (1996) Proc Natl Acad Sci USA 93:7279-7282].

SHaPrP90-231 in $\alpha$-helical, $\beta$-sheet and random coil conformations were covalently attached to glutaraldehyde activated polystyrene plates and incubated with serially diluted primary antibody. The amount of IgG reacting with each conformation of SHaPrP90-231 was determined either directly with Eu-labeled 3F4 IgG, or indirectly with europium labeled anti-rabbit or anti mouse antibody, according to usual protocols and the total signal was measured by time-resolved, dissociation-enhanced fluorescence. For the assay development were selected antibodies with the signal ratio of denatured versus $\beta$-sheet conformation of SHaPrP90-231 equal or higher than 4.

## EXAMPLE 4

COMPETITIVE AND DIRECT ASSAY FORMAT

Purified recombinant SHaPrP90-231, refolded into $\alpha$-helical or $\beta$-sheet conformation, was diluted into 5% (w/v) brain homogenate obtained from $PrP^{0/0}$ mouse and containing no prion protein. The brain homogenate was made by three 30 sec bursts in PowerGen homogenizer equipped with plastic disposable probe in TBS, pH 7.4 containing protease inhibitors cocktail (1 mM PMSF, 2 $\mu$g/ml of Aprotinin, and 2 $\mu$g/ml of Leupeptin) and spun at 5°C for 5 min at 500 G in a desktop centrifuge. The resulting supernatant was diluted 1:1 in TBS with final 4% (w/v) Sarcosyl and homogenized again by three 30 sec bursts in a PowerGen homogenizer. Next, the homogenate was spiked with different dilutions of recombinant SHaPrP90-231 in $\alpha$-helical or $\beta$-sheet conformations.

In a typical competitive assay, the analyte PrP in different conformations is preincubated with europium labeled 3F4 IgG and then transferred to the polystyrene plate coated with recombinant ShaPrP90-231 in SDS-denatured state. The results for analyte SHaPrP90-231 in $\alpha$-helical and denatured state (Figure 2) indicate marked difference in both available binding sites and affinity of europium-labeled 3F4 IgG with different conformations of prion protein.

In direct assay, each sample of dilution curve was divided into two aliquots: (1) untreated and designated native; (2) mixed with final 4M Gdn HCl and heated for 5 min at 100°C and designated denatured. Both samples were diluted 20-fold by $H_2O$ and aliquots loaded on polystyrene plate activated with glutaraldehyde. The plates, incubated overnight at 5°C, were blocked with TBS, pH 7.8, containing 0.5% BSA (w/v) and 6% Sorbitol (w/v). In the next step, they were washed three time with TBS, pH 7.8 containing 0.05% (v/v) of Tween® 20 and incubated with europium-labeled antibodies listed above. The plates were developed after an additional 7 washing steps in enhancement solution provided by the europium label supplier (Wallac Inc., Turku, Finland) and signal counted on DELFIA 1234 Fluorometer (Wallac Inc., Turku, Finland).

## EXAMPLE 5

DIFFERENTIAL TEST FOR VARIOUS CONFORMATIONS OF SHaPrP90-231

The parameters obtained from direct assay with Eu-labeled 3F4 IgG were plotted as a function of the concentration. The results obtained with SHaPrP90-231 in $\alpha$-helical conformation (Figure 3) indicate relatively small difference between signal of $\alpha$-helical and denatured protein. The sensitivity limit for denatured PrP in the presence of 5% brain homogenate is $\leq$ 1 ng/ml and linearity range over 3 orders of magnitude. In the experiments with the $\beta$-sheet form of SHaPrP90-231 (Figure 4), Eu-labeled 3F4 IgG bind strongly to a denatured form of the protein. In contrast, the reactivity with the native $\beta$-sheet form of the protein only marginally exceeded the background even at high protein concentrations. When the results are expressed as a ratio of the fluorescence of denatured versus native states of the prion protein (Figures 3 and 4), the ratio for $\alpha$-helical conformation is 1-1.8 and for recombinant SHaPrP90-231 in $\beta$-sheet conformation is 5-50.

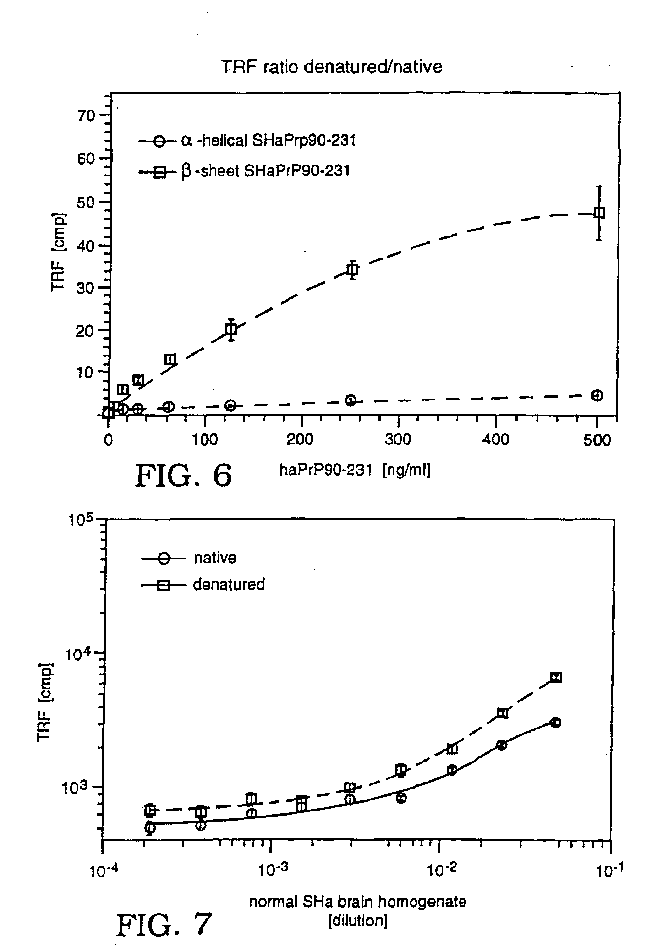

This effect was further utilized to analyze PrP samples of unknown conformation where the small increase in signal of Eu-labeled 3F4 IgG after denaturation of PrP is a characteristic of $\alpha$-helical conformation. By contrast, the large increase in the signal above the expected change for $\alpha$-helical conformation is diagnostic of the PrP90-231 in $\beta$-sheet conformation (Figures 3 and 4). The results are expressed in two different forms: (1) as a ratio (Figure 6), where index $\leq$ 1.8 for recombinant SHaPrP90-231 indicates that the protein was originally in all $\alpha$-helix conformation and index >1.8 indicates presence of $\beta$-sheet conformation; (2) as a formula shown herein and exemplified in Example 11, where the excess increase of signal above that expected for $\alpha$-helical conformation is proportionate to the amount of SHaPrP90-231 in $\beta$-sheet conformation.

**EXAMPLE 6**

QUANTITATIVE ASSAY FOR RECOMBINANT SHaPrP90-231 AND PrP$^C$

The input/output calibration for denatured ShaPrP90-231 in both α-helical and β-sheet conformations was linear within three orders of magnitude, and provide a high degree of confidence (Figure 5) for the assay. Also assayed was PrP$^c$, serially diluted into PrP$^{0/0}$ mouse homogenate which provided results with high degree of confidence within linearity range of 3 orders of magnitude. Next, the assay was calibrated by purified infectious PrP$^{Sc}$ in the presence of 5% PrP$^{0/0}$ brain homogenate. The calibration with PrP$^{Sc}$ provided a linear response within similar range.

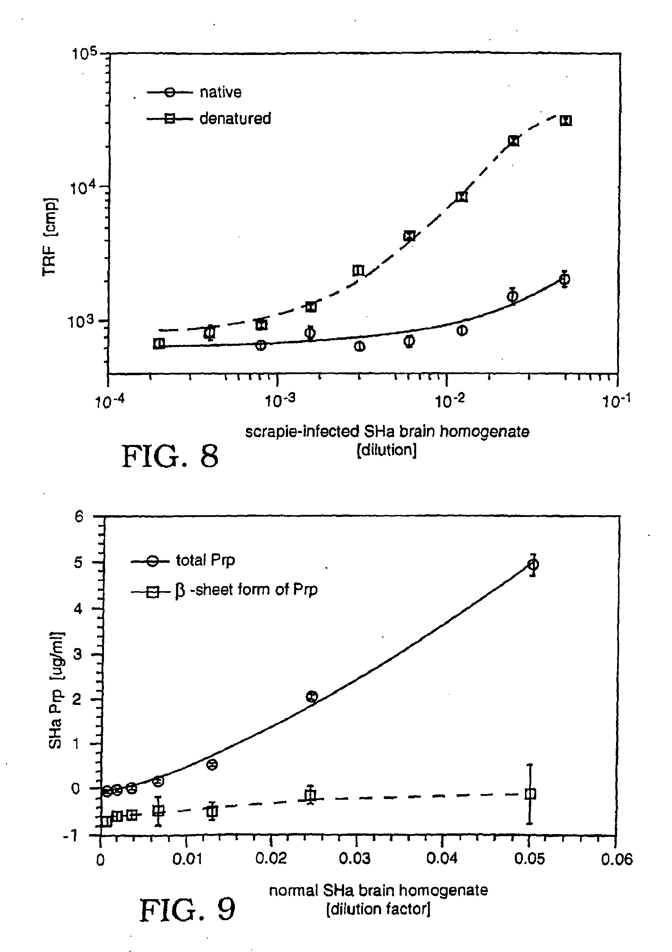

Using the differential method, the ratio between the signal of denatured versus native brain PrP$^c$ was for Eu-labeled monoclonal 3F4 IgG 2.2 (Figure 7), for polyclonal N12 and P3 1.0. The calibration for PrP$^{Sc}$ gave ratio ≥20 (Figure 8) for 3F4 Eu-labeled IgG and >4 for N12 and P3 antibodies. By using the formulae developed shown herein, all PrP$^c$ was in full range in α-helical conformation. In contrast, the calculated amount of PrP$^{Sc}$ in infectious, β-sheet conformation was as anticipated close to the total amount of prion protein.

**EXAMPLE 7**

DIAGNOSIS OF PRION DISEASE BASED ON INCREASE OF TOTAL PRION PROTEIN ABOVE PHYSIOLOGICAL PRP$^C$ LEVELS

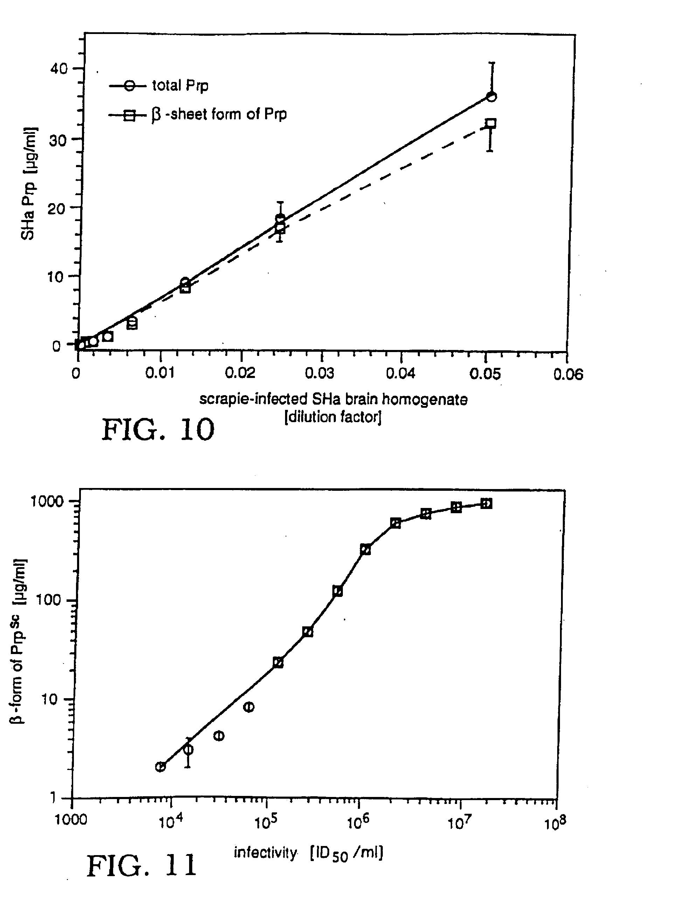

Accurate quantitative measurement of total PrP by exclusively evaluating the signal of denatured sample gave an average value of PrP$^c$ in 5% normal Syrian Hamster brain homogenates of 5.0 ± 0.2 μg/ml (Mean ± SEM) (Figure 9). The serial dilution of scrapie (Strain Sc237)-infected hamster brain homogenate into PrP$^{0/0}$ mouse brain homogenate gave values of total PrP 36.0±4 μg/ml (Figure 10) with broad linearity of the measurement. Because the only known condition for accumulation of prion protein is the accumulation of the infectious PrP$^{Sc}$ form, the increased levels of total PrP in the tested sample is indicative of the presence of prions. This prion assay may be used: (1) in direct mode in brain, tissue samples, body fluids or pharmaceuticals after determination of normal control values; or (2) in indirect mode by detecting the elevation of total PrP in the brain of experimental animal intracerebrally inoculated with tested tissue, body fluid or pharmaceutical sample suspected of containing prions.

**EXAMPLE 8**

DIAGNOSIS OF PRION DISEASE BASED ON INCREASE OF THE SIGNAL RATIO BETWEEN DENATURED VS NATIVE PRP ("PRION INDEX")

The ratio between antibody affinity for denatured versus native Syrian hamster brain PrP$^c$ protein is in the broad concentration range for Eu-labeled 3F4 IgG between 0.8-2.2. The ratio in the scrapie infected brain is through the full linearity range above those values (Figure 11). The "prion index" gives a relative indicator of the presence of infectious PrP$^{Sc}$ and therefore prions. This mode of prion assay is being used: (1) in direct mode in brain, tissue samples, body fluids or pharmaceuticals after determination of the index for normal controls; or (2) in indirect mode by detecting the elevated denatured/native prion protein index in the brain of experimental animals intracerebrally inoculated with tested tissue, body fluid or pharmaceutical sample suspected from containing prions.

**EXAMPLE 9**

DIAGNOSIS OF PRION DISEASE FROM DIFFERENTIAL ASSAY BY CALCULATING PrP$^{Sc}$ CONTENT

By using the direct assay and formulae provided here, there is no detectable amount of β-sheet form of PrP$^{Sc}$ in normal brain homogenate. Conversely, most of the total PrP in scrapie-infected hamster brain was due to the accumulation of PrP$^{Sc}$ (Figures 9 and 10). The correlation between the prion titer and PrP$^{Sc}$ calculated from the formula has a broad linearity and sensitivity cutoff of $\sim 10^3$ ID$_{50}$/ml (Figure 11). The formula gives a quantitative indicator of the presence of PrP protein in abnormal conformation which quantitatively correlates with prion titer. This mode of prion assay is used: (1) in direct mode in brain, tissue samples, body fluids or pharmaceuticals; or (2) in indirect mode, by calculating PrP$^{Sc}$ content in the brain of experimental animals intracerebrally inoculated with tested tissue, body fluid or pharmaceutical samples suspected for containing prions. After establishing a calibration curve between PrP$^{Sc}$ and prion titer, it is possible to estimate the titer directly from PrP$^{Sc}$ content.

## EXAMPLE 10

THE MEASUREMENT OF A-HELIX-TO-B-SHEET CONVERSION OF PrP PROTEIN IN VITRO TO SCREEN PRION GENERATION DE NOVO AND POTENTIAL DISEASE THERAPEUTICS

The aliquots of a 100 μg/ml solution of the α-helical form of recombinant SHaPrP90-231, or SHaPrP29-231, or corresponding recombinant or synthetic peptides of the prion protein are incubated in 20 mM Na acetate buffer, pH 5.5, for 24 hrs at 37°C with $10^{-3}$ - $10^{-6}$ M concentrations of glycerol, cyclodextrins, heparin, heparin sulfate, Congo Red, cholesterol ester, dimyristoyl phosphatidylcholine. The samples are then divided into two aliquots: (1) untreated, designated native; (2) mixed with final 4M Gdn HCl and heated for 5 min at 100°C, designated denatured. Both samples are diluted 20-fold by $H_2O$ and aliquots loaded on polystyrene plate activated with glutaraldehyde. The plates, incubated overnight at 5°C, were blocked with TBS, pH 7.8, containing 0.5% BSA (w/v) and 6% Sorbitol (w/v). In the next step, they were washed three time with TBS, pH 7.8 containing 0.05% (v/v) of Tween® 20 and incubated with europium-labeled 3F4 IgG. The plates were developed after additional 7 washing steps in enhancement solution provided by the europium label supplier (Wallac Inc., Turku, Finland) and signal counted on DELFIA 1234 Fluorometer (Wallac Inc., Turku, Finland).

The degree of conversion from α-helical to β-sheet conformation of PrP is calculated from the "prion index" or alternatively from the formulae provided herein. Some compounds which inhibit the conversion by apparently stabilizing the native-like conformation of prion protein may have therapeutic potential in vivo.

## EXAMPLE 11

The assay method is demonstrated on the following example with scrapie-infected Syrian hamster brain homogenate, diluted 4-fold into $PrnP^{0/0}$ mouse brain homogenate:

a) Each plate is calibrated with an inner standard consisting from five dilution points of denatured SHaPrP90-231. The time-resolved fluorescence (TRF) of total PrP is developed with Eu-labeled 3F4 IgG and the time-resolved fluoresence values are plotted as a function of PrP concentration (Figure 4). The data are fit within a linear or polynomial equation using the least square method and best function is selected for the calculation of denatured PrP:

$$PrP\ [\mu g/ml] = -0.22935 + 0.00026567*[TRF] +$$

$$0.0000000012255*[TRF]^2 \tag{1}$$

b) On the rest of the plate, native and denatured aliquots of scrapie-infected Syrian hamster brain homogenate, diluted 4-fold, and crosslinked to the plastic support were incubated with Eu-labeled 3F4 IgG. The total PrP content is calculated according to the above formula from the fluorescence signal of denatured sample:

| scrapie infected brain homogenate concentration [%] | native TRF [cpm] | denatured TRF [cpm] | $PrP^{c+Sc}$ [μg/ml] |
|---|---|---|---|
| 5 | 4214 | 109814 | 43.7 |
| 1.25 | 1381 | 30804 | 9.1 |
| 0.3125 | 1070 | 11240 | 2.9 |

c) The ratio of the fluorescence signals between denatured and native samples is calculated:

| scrapie infected brain homogenate concentration [%] | native TRF [cpm] | denatured TRF [cpm] | denatured/ native ratio |
|---|---|---|---|
| 5 | 4214 | 109814. | 26.1 |
| 1.25 | 1381 | 30804 | 22.3 |

(continued)

| scrapie infected brain homogenate concentration [%] | native TRF [cpm] | denatured TRF [cpm] | denatured/ native ratio |
|---|---|---|---|
| 0.3125 | 1070 | 11240 | 10.5 |

The normal value of PrP$^c$ determined from normal hamster brain homogenate is 2.2; the values over 2.2 are considered abnormal and indicate the presence of PrP$^{Sc}$.

d) The excess of fluoresence signal over that expected for $\alpha$-helical PrP in the transition from native to denatured state is a measure of the amount of PrP$^{Sc}$ and is calculated according the formulae provided:

$$\Delta F_{\beta n \to d} = F_d - (F_n * f_{\alpha\, n \to d}) \tag{2}$$

where f = is the maximum value of the factor for the fluorescence signal in the transition from native to denatured state of PrP$^c$; $F_d$ is the fluorescence of denatured sample; and $F_n$ is the fluorescence of native sample. The amount of PrP$^{Sc}$ is then calculated from $\Delta F_{\beta n \to d}$ and equation (1):

| scrapie infected brain homogenate concentration (%) | $\Delta$TRF$_{\beta n-d}$ [cpm] | PrP$^{Sc}$ [$\mu$g/ml] |
|---|---|---|
| 5 | 100543.2 | 38.9 |
| 1.25 | 27765.8 | 8.1 |
| 0.3125 | 8886 | 2.2 |

The positive value calculated for the $\beta$-sheet form of prion protein indicates the presence of PrP$^{Sc}$.

## EXAMPLE 12

STANDARDS OF SOLUBLE ($\alpha$-HELICAL) AND INSOLUBLE ($\beta$-SHEET) FORMS OF $\beta$A4 PROTEIN

Soluble forms of $\beta$A4 (1-40) and $\beta$A4 (1-42) were obtained from Bachem (Torrance, CA). One portion of the fresh lyophilized peptide was solubilized in PBS, pH 7.4, containing 20% (v/v) of HFIP (hexafluoroisopropanol; 1,1,1,3,3,3-hexafluoro-2-propanol) or 1% (w/v) SDS (sodium dodecylsulfate) at final protein concentration 50 $\mu$M; this protein was designated "soluble" and was stored at -80°C until use. A second portion of the peptide was resuspended in PBS, pH 7.4 at the final concentration $\geq$350 $\mu$M and incubated for 72 hrs at 37°C. This portion of the protein was designated "insoluble" and was stored until use at -80°C.

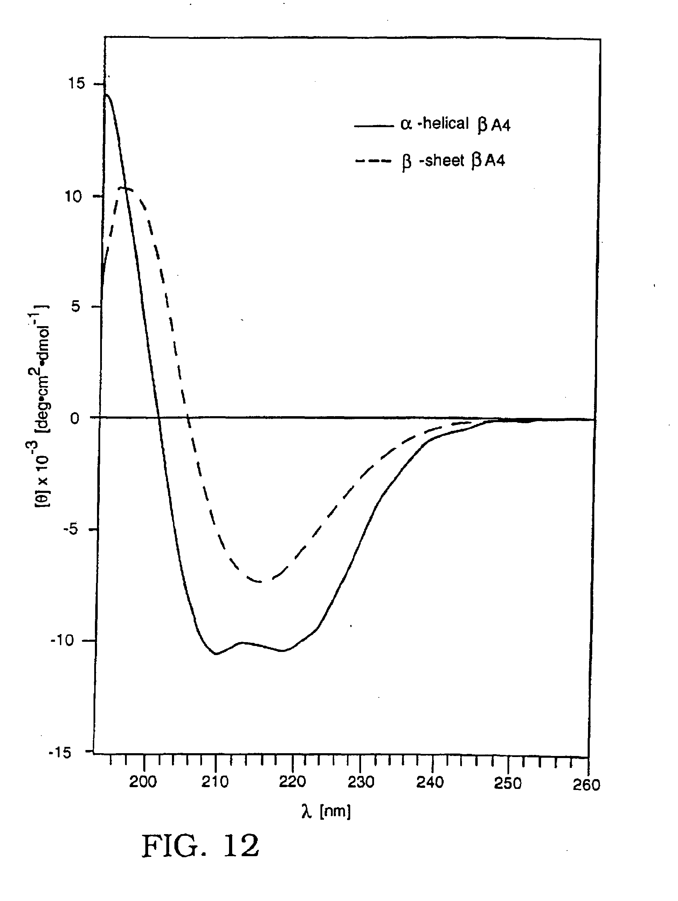

Both proteins were used as a standards for assay development and to establish the sensitivity and linearity range. The CD (circular dichroism) spectroscopy (Figure 12) demonstrated that soluble protein has an $\alpha$-helical conformation (see the solid line of Figure 12). In contrast, the $\beta$A4 protein treated for 72 hrs at 37°C has fully converted into $\beta$-sheet conformation (see the dashed line of Figure 12).

SELECTION, LABELING AND DETECTION METHOD OF ANTIBODIES USED IN THE ASSAY

The protocols and methods of antibody production and characterization are generally described elsewhere [Harlow and Lane (1988) supra:726]. The data described in this and following examples were generated with monoclonal antibody 6F3D, developed against synthetic peptide corresponding to the sequence of human $\beta$A4 protein (Research Diagnostics Inc., Flanders, NJ). However, polyclonal antibodies made against synthetic analog of $\beta$A4 protein might also be used. Recombinant Fab recognizing denatured forms of $\beta$A4 protein might also be used.

$\beta$A4 protein in $\alpha$-helical, $\beta$-sheet and random coil conformations were covalently attached to the glutaraldehyde activated polystyrene plates and incubated with serially diluted primary antibody. The amount of IgG reacting with each conformation of $\beta$A4 was determined with europium labeled anti-rabbit or anti mouse antibody according usual protocols and the total signal was measured by time-resolved, dissociation-enhanced fluorescence. Antibodies with the signal ratio of denatured versus $\beta$-sheet conformation of $\beta$A4 equal or higher than 2 were selected to develop the assay.

## DIRECT AND COMPETITIVE ASSAY FORMAT

In direct assay, each sample of dilution curve of α-helical and β-sheet forms of βA4 protein was divided into two aliquots: (1) untreated (designated native); and (2) mixed with final 4M Gdn·HCl/1% Sarcosyl and heated for 5 min at 100°C (designated "denatured"). Both samples were diluted 20-fold by $H_2O$ and aliquots loaded on polystyrene plate activated with 0.2% glutaraldehyde for 2 hrs. The plates, incubated overnight at 5°C, were blocked with TBS, pH 7.8, containing 0.5% BSA (w/v) and 6% Sorbitol (w/v). The samples were then washed three time with TBS, pH 7.8 containing 0.05% (v/v) of Tween® 20 and incubated with primary antibodies against βA4 protein (6F3D, Research Diagnostics Inc., Flanders, NJ). The samples were washed and then developed with europium-labeled secondary antibodies against mouse IgG (Wallac Inc., Turku, Finland). The plates were developed after an additional 7 washing steps in enhancement solution (Wallac Inc., Turku, Finland) and signal counted on DELFIA 1234 Fluorometer (Wallac Inc., Turku, Finland). The results for analyte indicate marked difference in both available binding sites and affinity anti-βA4 IgG with different conformations of βA4 protein.

The method could be carried out using a competitive assay, where the analyte βA4, in different conformations is preincubated with anti-βA4 IgG and then transferred to the polystyrene plate coated with synthetic βA4 protein in Gdn·HCl-denatured state.

## DIFFERENTIAL TEST FOR VARIOUS CONFORMATIONS OF βA4

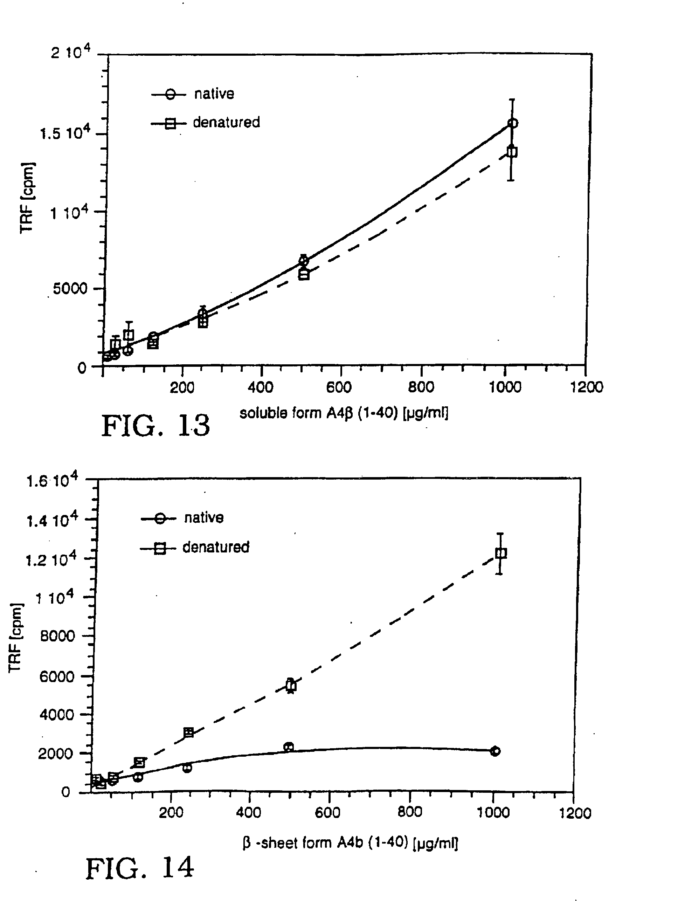

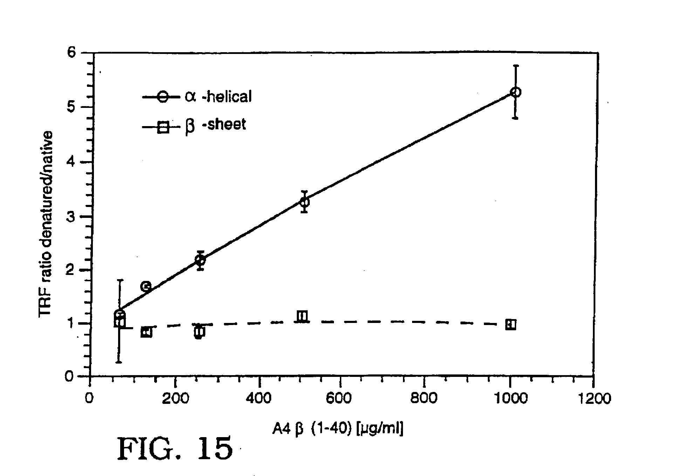

The parameters obtained from direct assay with 6F3D anti-βA4 IgG were plotted as a function of the concentration. The results obtained with βA4 in β-helical conformation (Figure 13) show a large difference between the signal of β-sheet and denatured protein. The sensitivity limit for denatured βA4 is ≤ 1 μg/ml and the linearity range is over 2 orders of magnitude. In the experiments with the α-helical form of βA4 (Figure 14), 6F3D IgG binds equally strongly to both native and denatured forms of the protein. In contrast, the reactivity with the native β-sheet form of the protein only marginally exceeded the background even at high protein concentrations. When the results are expressed as a ratio of the fluorescence of denatured versus native states of the βA4 (Figure 15), the ratio for α-helical conformation is ≤ 1 and for βA4 in β-sheet conformation is in a given concentration range ≥ 1.5.

This effect was further utilized to analyze βA4 samples of unknown conformation where the small increase in signal of Eu-labeled 3F4 IgG after denaturation of βA4 is a characteristic of α-helical conformation. By contrast, the large increase in the signal above the expected change for α-helical conformation is diagnostic of the in β-sheet conformation (Figure 15). The results may be expressed in two different forms: (1) as a ratio (Figure 15), where index ≤ 1.0 for βA4 indicates that the protein was originally in all α-helix conformation and index >1.5 indicates presence of β-sheet conformation; (2) per the formula shown above where the excess increase of signal above that expected for α-helical conformation is proportionate to the amount of βA4 in β-sheet conformation.

## EXAMPLE 13

### DIAGNOSIS OF ALZHEIMER'S DISEASE BASED ON INCREASE OF TOTAL βA4 PROTEIN ABOVE PHYSIOLOGICAL LEVELS

Accurate quantitative measurement of total βA4 by exclusively evaluating the signal of denatured sample is apparently more accurate than measurement of βA4 in native conformation (Figures 13 and 14). The normal value of the protein may hide a significant portion of the β-sheet form of βA4 protein due to the lower reactivity of this form with antibodies. Because the only known condition for accumulation of βA4 protein is the accumulation of the β-sheet form of βA4 in the brains of patients with Alzheimer's disease, the increased levels of total βA4 in the tested sample is indicative of the presence of pathogenic forms of βA4. This βA4 assay may be used in tissue, body fluids or pharmaceutical sample suspected of containing β-sheet forms of βA4.

### DIAGNOSIS OF ALZHEIMER'S DISEASE BASED ON INCREASE OF THE SIGNAL RATIO BETWEEN DENATURED vs. NATIVE βA4 ("βA4 AMYLOID INDEX")

The ratio between antibody affinity for denatured versus native human βA4 protein is in the broad concentration range for 6F3D IgG between 0.8-1.0. The ration for β-sheet βA4 is through the full linearity range above those values (Figure 15). The "βA4 amyloid index" gives a relative indicator of the presence of pathogenic insoluble β-sheet βA4. This mode of βA4 assay may be used in direct mode in brain, tissue samples, body fluids or pharmaceuticals after determination of the index for normal controls.

DIAGNOSIS OF ALZHEIMER'S DISEASE FROM DIFFERENTIAL ASSAY BY CALCULATING βA4 CONTENT

By using the direct assay and formula shown above, the amount of pathogenic forms of β-sheet βA4 protein can be calculated in brain, tissue samples, body fluids or pharmaceuticals.

**EXAMPLE 14**

THE MEASUREMENT OF α-HELIX-TO-β-SHEET CONVERSION OF βA4 PROTEIN IN VITRO TO SCREEN THE POTENTIAL DISEASE THERAPEUTICS

The aliquots of a 350 μM solution of the α-helical form of synthetic βA4 (1-40, or corresponding recombinant or synthetic peptides of the βA4 protein are incubated in PBS, pH 7.4, for 72 hrs at 37°C with $10^{-3}$ - $10^{-6}$ M concentrations of glycerol, cyclodextrins, heparin, heparin sulfate, Congo Red, cholesterol ester, dimyristoyl phosphatidylcholine. The samples are then divided into two aliquots: (1) untreated, containing 1% Sarcosyl and designated "native"; and (2) mixed with final 4M Gdn HCl/1% Sarcosyl and heated for 5 min at 100°C, designated "denatured". Both samples are diluted 20-fold by $H_2O$ and aliquots loaded on polystyrene plate activated with glutaraldehyde. The plates, incubated overnight at 5°C, were blocked with TBS, pH 7.8, containing 0.5% BSA (w/v) and 6% Sorbitol (w/v). In the next step, they were washed three time with TBS, pH 7.8 containing 0.05% (v/v) of Tween® 20 and incubated with 6F3D IgG and then with Eu-labeled anti-mouse antibody. The plates were developed after an additional 7 washing steps in enhancement solution and signal counted on DELFIA 1234 Fluorometer (Wallac Inc., Turku, Finland).
The degree of conversion from α-helical to β-sheet conformation of βA4 is calculated from the "amyloid index" (Figure 15) or alternatively from the formula shown above. Any compound which inhibits the conversion by stabilizing the α-helical conformation of βA4 protein may have therapeutic potential in vivo by preventing formation of mature amyloid.

**EXAMPLE 15**

STANDARDS OF NORMAL AND AMYLOID FORMS OF TTR

Soluble forms of TTR were obtained from Sigma Chemical Comp. One portion of the protein was solubilized in 100 mM KCl buffer, pH 7.4, at final protein concentration 0.5 mg/ml; this protein was designated "normal" and was stored at -80°C until use. The second portion of the protein was converted into amyloid as described [Lai, Colon et al. (1996) Biochemistry 35(20):6470-82]. Briefly, the protein was resuspended in 100 mM KCl buffer, containing 50 mM sodium acetate, pH 4.4, at protein concentration 0.2 mg/ml, and incubated for 72 hrs at 37°C. This portion of the protein was designated "amyloid" and was stored until use at -80°C. The turbidimetry and Congo Red binding assay verified the efficient conversion into amyloid [Lai, Colon et al. (1996) Biochemistry 35(20):6470-82]. Both proteins were used as standards for the assay development and to establish the sensitivity and linearity range.

DIRECT ASSAY FORMAT

The protocols and methods of antibody production and characterization are in general described elsewhere. The data described in this and following examples were generated with commercially available polyclonal antibody, developed against purified human TTR (Accurate Chemical and Scientific Corporation, Westbury, NY).
In the direct assay, each sample of protein taken from the dilution curve of normal and amyloid forms of TTR was divided into two aliquots: (1) untreated and designated "native"; (2) mixed with final 4M Gdn HCl/1% Sarcosyl and heated for 5 min at 100°C and designated "denatured." Both samples were diluted 20-fold by $H_2O$ and aliquots loaded on polystyrene plate activated with 0.2% glutaraldehyde for 2 hrs. The plates, incubated overnight at 5°C, were blocked with TBS, pH 7.8, containing 0.5% BSA (w/v) and 6% Sorbitol (w/v). In the next step, they were washed three times with TBS, pH 7.8 containing 0.05% (v/v) of Tween 20 and incubated with primary antibodies against TTR (Accurate Chemical and Scientific Corporation, Westbury, NY), washed and then developed with europium-labeled secondary antibodies against rabbit IgG (Wallac Inc., Turku, Finland). The plates were developed after an additional 7 washing steps in enhancement solution and signal counted on DELFIA 1234 Fluorometer (Wallac Inc, Turku, Finland).

## EXAMPLE 16

DIAGNOSIS OF SSA AND FAP BASED ON INCREASE OF THE SIGNAL RATIO BETWEEN DENATURED VS. NATIVE TTR ("TTR AMYLOID INDEX")

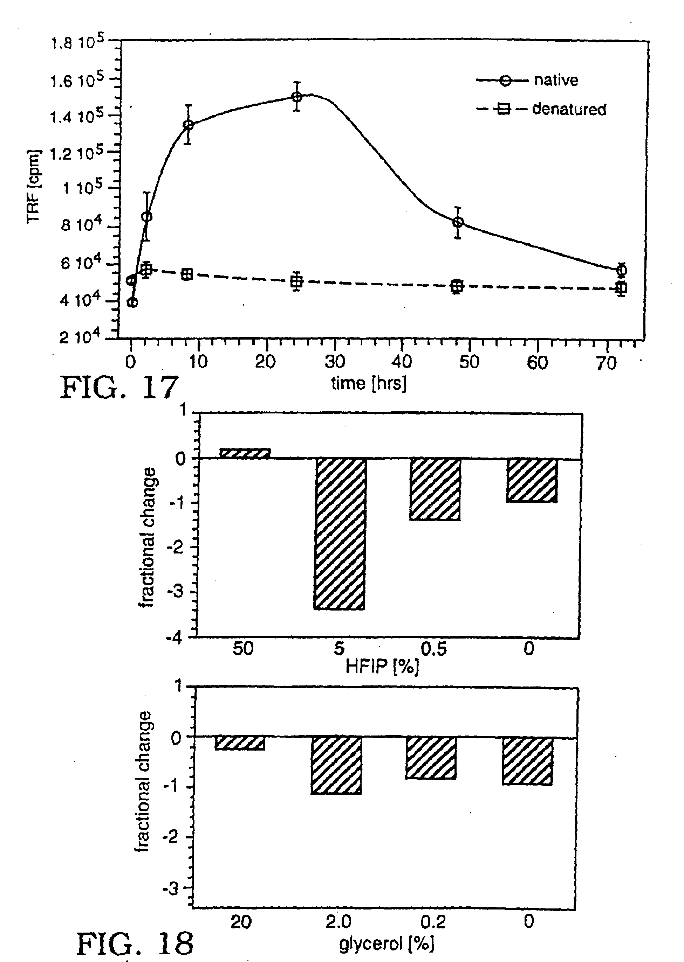

The ratio between antibody affinity for denatured versus native form of normal human TTR is in the broad concentration range for polyclonal antibody 1-3:3. The ratio for amyloid form of TTR is through the full linearity range between 0.7-1.0 (Figure 18). The "TTR amyloid index" gives a relative indicator of the presence of pathogenic, insoluble and amyloid-forming TTR. This mode of TTR assay is being used in direct mode in brain, tissue samples, body fluids or pharmaceuticals after determination of the index for normal controls.

DIAGNOSIS OF SSA AND FAP FROM DIFFERENTIAL ASSAY BY CALCULATING TTR AMYLOID CONTENT

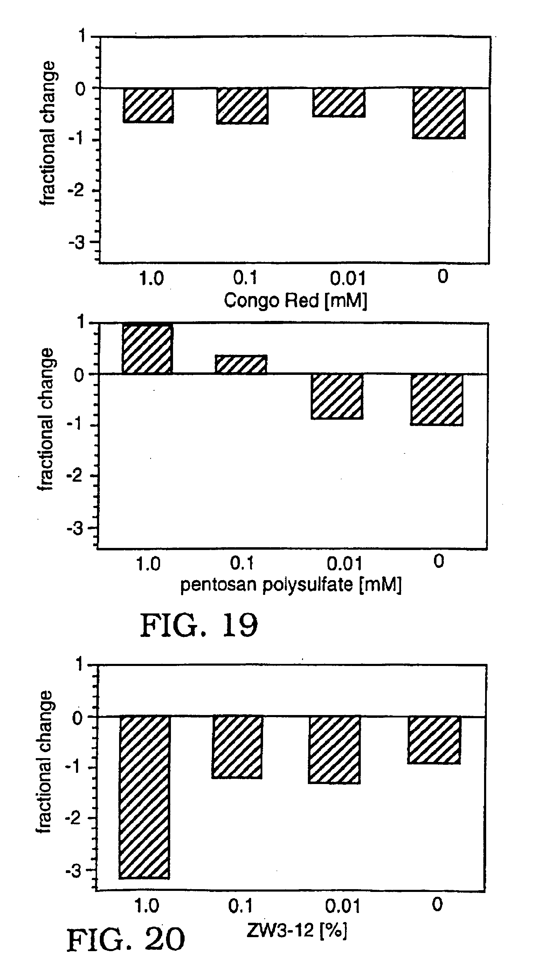

By using the direct assay formula (Figure 19), the amount of amyloid form of TTR can be calculated in peripheral nerve, tissue samples; body fluids or pharmaceuticals. In formula (Figure 19), the lower than expected increase of signal for normal conformation is proportional to the amount of TTR in amyloid conformation.

## EXAMPLE 17

THE MEASUREMENT OF NORMAL-TO-AMYLOID CONVERSION OF TTR IN VITRO TO SCREEN THE POTENTIAL DISEASE THERAPEUTICS

The aliquots of a 0.2 mg/ml solution of the normal form of synthetic TTR, or corresponding recombinant or synthetic peptides of the TTR are incubated in 100 mM KCl buffer, containing 50 mM of sodium acetate, pH 4.4 for 72 hrs at 37°C with $10^{-3}$ - $10^{-6}$ M concentrations of tested organic compounds. The samples are then divided into two aliquots: (1) untreated, containing 1% Sarcosyl and designated "native"; (2) mixed with final 4M Gdn HCl/1% Sarcosyl and heated for 5 min at 100°C, designated "denatured." Both samples are diluted 20-fold with $H_2O$ and aliquots loaded on polystyrene plate activated with glutaraldehyde. The plates, incubated overnight at 5°C, were blocked with TBS, pH 7.8, containing 0.5% BSA (w/v) and 6% Sorbitol (w/v). In the next step, they were washed three time with TBS, pH 7.8 containing 0.05% (v/v) of Tween® 20 and incubated with polyclonal anti-TTR antibody (Accurate Chemical and Scientific Corporation, Westbury, NY) and then Eu-labeled anti-rabbit antibody. The plates were developed after additional 7 washing steps in enhancement solution and the signal counted on DELFIA 1234 Fluorometer (Wallac Inc., Turku, Finland).

The degree of conversion from normal to amyloid conformation of TTR is calculated from the "amyloid index" (Figure 18) or alternatively from the formula (Figure 19). Any compound which inhibits the conversion by stabilizing normal conformation of TTR may have therapeutic potential *in vivo* by preventing formation of mature amyloid.

## EXAMPLE 18

TYPING OF PRION ISOLATES (STRAINS) IN SYRIAN HAMSTERS

Syrian hamsters (LVG/LAK) were infected by intracerebral injection of the following hamster adapted scrapie isolates, i.e., different groups of hamsters were infected with individual strains of prions as follows: Drowsy (Dy), 139H, Hyper (Hy), Me7, MT-C5, and Sc237. The animals were euthanized in terminal stages of disease and their brains immediately frozen and stored at -70°C. Brains were homogenized on ice by 3x30 sec strokes of PowerGen homogenizer (Fisher Scientific, Pittsburgh, PA) in PBS, pH 7.4, containing protease inhibitors cocktail (PMSF 5mM, Aprotinin and Leupeptin 4 μg/ml). Resulting 10% (w/v) homogenates were spun for 5 min at 500 g at table top centrifuge. The supernatant was mixed 1:1 with 4% Sarcosyl in PBS, pH 7.4 and divided into two aliquots: (1) untreated and designated native; (2) mixed with a final concentration of 4M Gdn Hcl and heated for 5 min at 80-100°C and designated denatured. Both samples were diluted 20-fold by $H_2O$ and aliquots loaded on polystyrene plate activated for 1 hr with 0.2% glutaraldehyde in PBS. The plates, incubated overnight at 5°C, were blocked with TBS, pH 7.8, containing 0.5% BSA (w/v) and 6% Sorbitol (w/v). In the next step, they were washed three times with TBS, pH 7.8 containing 0.05% (v/v) of Tween 20 and incubated for 2 hrs with Europium-labeled monoclonal antibody 3F4. The plates were developed after an additional 7 washing steps in enhancement solution provided by the Europium label supplier (Wallac Inc., Turku, Finland) and signal counted on DELFIA 1234 Fluorometer (Wallac Inc., Turki, Finland). The PrP$^{Sc}$ content and "prion index" (ratio of antibody binding to denatured:native PrP protein) were calculated as described in Examples 11 and 8 and plotted in xy coordinates as shown in Figure 25. Other samples tested and resulting in the same calculations could be presumed to have the same strain of prion therein.

The instant invention is shown and described herein in what is considered to be the most practical, and preferred embodiments. It is recognized, however, that departures may be made therefrom, which are within the scope of the invention, and that obvious modifications will occur to one skilled in the art upon reading this disclosure.

**Claims**

1. A method for determining the presence of a $PrP^{Sc}$ form of a PrP protein in a sample comprising a first $PrP^{c}$ conformation of the protein and a second $PrP^{Sc}$ conformation of the protein, the method comprising:

   contacting a first portion of the sample with a binding partner, said binding partner having a higher affinity for the $PrP^{c}$ conformation than the $PrP^{Sc}$ conformation, and determining a first concentration;
   treating a second portion of the sample to change the $PrP^{Sc}$ conformation into a conformation with a higher binding affinity for the binding partner;
   contacting the treated second portion of the sample with the binding partner to determine a second concentration;
   adjusting the second concentration to provide an adjusted concentration which adjustment compensates for increased affinity of the $PrP^{c}$ conformation for the binding partner resulting from the treating; and
   comparing the first concentration with the adjusted concentration to determine the presence of protein in the $PrP^{Sc}$ conformation.

2. The method of claim 1, wherein the sample is obtained from an animal not exhibiting symptoms of disease;
   wherein the first concentration and the second concentration are determined using time-resolved, disassociation-enhanced fluorescence; and
   further wherein the $PrP^{Sc}$ conformation of the protein is present in the sample in a concentration of $1 \times 10^3$ particles/ml or less.

3. The method of claim 1, wherein said treating comprises subjecting said second portion of the sample to chemical denaturation, sufficient to convert at least 2% of any $PrP^{Sc}$ protein to said binding form;
   wherein said binding partner comprises a labelled antibody having an affinity for said $PrP^{c}$ conformation at least four times higher than its affinity for said $PrP^{Sc}$ conformation.

4. A method for determining the presence of a $PrP^{Sc}$ form of a PrP protein in a sample comprising a $PrP^{c}$ conformation of the protein and a $PrP^{Sc}$ conformation of the protein, the method comprising:

   treating the sample to convert the $PrP^{Sc}$ conformation of the protein into a binding conformation having an affinity for a binding partner higher than the $PrP^{Sc}$ conformation;
   contacting the treated sample with the binding partner to determine a concentration;
   adjusting the concentration to provide an adjusted concentration which compensates for increased affinity of the $PrP^{c}$ conformation of the protein to the binding partner resulting from the treating; and
   comparing said adjusted first concentration to a known concentration selected from the group consisting of a control concentration and a predetermined standard concentration to determine the presence of the protein in the $PrP^{Sc}$ conformation in the sample.

5. The method of claim 4, wherein said binding partner comprises a labelled antibody, and wherein the concentration is determined using flow cytometry;
   wherein the adjusted concentration is compared to a known concentration determined from a treated non-infected control sample or compared to a known concentration predetermined from a treated sample from a non-infected population;
   further wherein the protein in the $PrP^{Sc}$ conformation is present in the sample in a concentration of $1 \times 10^3$ protein molecules or less per ml and wherein the protein in the $PrP^{c}$ conformation is present in the sample in a concentration of $1 \times 10^6$ protein molecules or more per ml.

6. A method for identifying a compound having a therapeutic activity against a prion-mediated disease, said prion-mediated disease **characterized by** a PrP protein having a $PrP^{c}$ form and $PrP^{Sc}$ form which differ in conformation, said $PrP^{Sc}$ form being associated with said prion-mediated disease, said method comprising:

   providing an animal susceptible to a prion-mediated disease;

administering a test compound to said animal;

inducing said prion-mediated disease;

obtaining a sample from said animal;

contacting a first portion of sample containing said protein with a binding partner, said binding partner having a higher affinity for said $PrP^c$ form of said protein than said $PrP^{Sc}$ form, and determining a first concentration;

treating a second portion of the sample to convert said $PrP^{Sc}$ form into a binding form having a higher affinity for said binding partner;

contacting the treated second portion with the binding partner to determine a second concentration;

adjusting the second concentration to provide an adjusted concentration which compensates for increased affinity of the protein in the $PrP^{Sc}$ form for the binding partner resulting from the treating; and

comparing the first concentration with the adjusted concentration to determine the concentration of the protein in the $PrP^{Sc}$ form and deducing the effect of the test compound on the concentration of protein in the $PrP^{Sc}$ form.

7.  A method of determining a strain of a $PrP^{Sc}$ protein in a sample comprising:

determining the concentration of a $PrP^{Sc}$ conformation of a protein in a sample;

treating the sample in a manner so as to change the $PrP^{Sc}$ conformation of the protein to a conformation having a higher binding affinity for a binding partner than the $PrP^{Sc}$ conformation;

determining the effect of the treating of the protein on the $PrP^{Sc}$ conformation; and

comparing the determined effect with a known standard effect for a known strain of $PrP^{Sc}$ at a known concentration and thereby deducing the strain of the $PrP^{Sc}$ conformation of the protein is the sample.

8.  The method of claim 7, wherein the concentration of the pathogenic conformation and effect by treatment are determined using a labled antibody as the binding partner and using time-resolved, dissociation-enhanced fluorescence;

wherein the $PrP^{Sc}$ is treated with proteinase K.

9.  The method of any one of claims 1, 2, 4, 6 and 7, wherein the binding partner is an antibody.

10. The method of claim 3, wherein said antibody has an affinity for said $PrP^c$ conformation at least ten times higher than its affinity for said $PrP^{Sc}$ conformation.

11. The method of claim 3, wherein said antibody has an affinity for said $PrP^{Sc}$ conformation at least thirty times higher than its affinity for said $PrP^{Sc}$ conformation.

12. The method of claim 9, wherein said antibody has an affinity for said $PrP^c$ conformation at least four times higher than its affinity for said $PrP^c$ conformation.

13. The method of claim 9, wherein said antibody has an affinity for said $PrP^c$ conformation at least thirty times higher than its affinity for said $PrP^{Sc}$ conformation.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

FIG. 22

FIG. 23

a)  $F_n = F_{nN} + F_{nA}$  $\longrightarrow$ $F_{nN} = F_n - F_{nA}$

b)  $F_d = F_{dN} + F_{dA}$

FIG. 24

$$\Delta F_{n \longrightarrow d} = \Delta F_{Nn \longrightarrow d} + \Delta F_{An \longrightarrow d}$$

$$\Delta F_{An \longrightarrow d} = F_d - \Delta F_{Nn \longrightarrow d}$$

$$[TTR_A] \sim \quad \Delta F_{An \longrightarrow d} = (F_n * f_{Nn \longrightarrow d}) - F_d$$

FIG. 25